# EUROPEAN PATENT APPLICATION

(11) **EP 4 800 692 A2**
(43) Date of publication of application: **02.09.2026**
(21) Application number: 26180404.1
(22) Date of filing: 17.03.2021
(51) Int. Cl.: G16B 50/50

(54) **METHOD AND SYSTEM FOR THE EFFICIENT DATA COMPRESSION IN MPEG-G**

(30) Priority: 15.04.2020 EP 20169717
(62) Document number(s) of the earlier application(s) in accordance with Art. 76 EPC: 21711587.2 / 4 136 640
(71) Applicant: Koninklijke Philips N.V., 5656 AE Eindhoven (NL)
(72) Inventor: ALBERTI, Claudio, 1015 Lausanne (CH); RAVASI, Massimo, 1015 Lausanne (CH); RIBECA, Paolo, Edinburgh, EH9 3FD (GB)
(74) Representative: Philips Intellectual Property & Standards

(57) **Abstract**

A computer-implemented method for
the storage or transmission of a representation of genome sequencing data in a genomic file format comprising annotation data associated with said genome sequencing data, said genome sequencing data comprising reads of sequences of nucleotides, said method comprising the steps of:
aligning (10) said reads to one or more reference sequences thereby creating aligned reads, classifying (14) said aligned reads according to classification rules based on mapping of said aligned reads on said one or more reference sequences, thereby creating classes of aligned reads (18), entropy encoding said classified aligned reads as a multiplicity of blocks of descriptors, structuring said blocks of descriptors with header information thereby creating Access Units of a first sort (119) containing genome sequencing data,
said method further comprising encoding annotation data (12) into different Access Units of a second sort (122) and indexing data into a master annotation index (MAI, 123, 211), wherein said indexing data represent an encoded form of annotation string data obtained by employing at least one compressed string indexing algorithm (28) on said annotation string data (212), and wherein said MAI associates encoded annotation strings with said access units of a second sort, said method further comprising jointly coding said access units of first sort, of second sort and said MAI.

## Description

### TECHNICAL FIELD

The present invention relates to the field of data compression of MPEG-G.

MPEG, Moving Picture Experts Group (MPEG) is a working group of data compression experts that was formed by ISO and IEC to set standards for audio and video compression and transmission.

This group has been developing standards for video efficient video compression since the early 90ies. The technology of MPEG essentially consists into the reduction of the entropy of the video and audio source data such that higher compression ratio can be achieved for efficient storage and transmission. Since there is a great expertise of data compression within the MPEG groups of expert, it was decided to develop a standard for the compression of genomic information to overcome the limitations of the solution present in the art (e.g. CRAM and BAM file formats).

Therefore, even if MPEG-G relates to the compression of genomic data, the main idea of exploitation of data redundancies is taken from the field of video and audio compression that is the closest technical field to the present application.

This invention in fact applies syntax elements construction for genomic data in a similar manner as the syntax elements are applied to the compression of video and audio data in MPEG.

Given the fact, however, that the genomic data are quite different from the audio and video data, the data classification and the syntax elements are different from those used in the MPEG video and audio standards: in fact the redundancies present in the genomic data have to exploited and these are different from the multimedia data.

The present invention therefore deals with the compression of genomic data in an efficient manner in order to obtain a file of reduced size and easy to be randomly accessed also in the compressed domain.

The present invention builds onto the encoding and decoding methods, systems and computer programs disclosed in the patent applications WO 2018/068827A1, WO 2018/068828A1, WO 2018/068829A1, WO 2018/068830A1, whose disclosures related to entropy coding of genomic data may be essential for the understanding of some aspects of the present invention; the disclosure of the aforementioned documents is therefore considered as incorporated by reference in the present invention.

This disclosure provides a novel method of representation of annotations and metadata associated to genome sequencing data which reduces the utilized storage space, provides a single syntax for several metadata formats and improves data access performance by providing new indexing functionality which is not available with known prior art methods of representation.

The method disclosed in this invention provides higher compression ratios for genome sequencing data and associated annotations by:
- representing said genome sequencing data and associated annotations in terms of a syntax of numeric and textual descriptors as defined in this disclosure
- compressing separately non-indexed descriptors from indexed textual descriptors
- applying to non-indexed descriptors transformations such as differential coding, run-length coding, bytes separation, and entropy coders such as CABAC, Huffman Coding, arithmetic coding, range coding
- applying compressed full-text string indexing algorithms such as compressed string pattern matching data structures, compressed suffix arrays, FM-indexes, and hash tables to indexed textual descriptor by eliminating the redundancy of having both an index and a compressed payload as done by existing methods.

The advantage of compressing separately non-indexed descriptors from indexed textual descriptors is that these 2 classes of data, once separately grouped, show a lower entropy than when they are coded together, therefore higher compression ratio can be achieved.

By using compressed full-text string indexing algorithms, the method described in this invention eliminates the need to have both a compressed payload of genomic information and an index of said information to support selective access, therefore reaching better compression ratios. The compressed full-text string indexing algorithms is at the same time an index and the compressed information and can be used both to perform selective access and to retrieve the desired information by decompression. This invention overcomes the need to have both an index and a compressed payload as currently required by existing solutions in the art.

The method also allows to hierarchically describe, and store in compressed form, concepts related to genomic annotation which were previously unrelated. This makes it possible to encode relations between such concepts that could not be described previously, thus allowing novel ways of describing and interchanging data.

### BACKGROUND

Genomic or proteomic information generated by DNA, RNA, or protein sequencing machines is transformed, during the different stages of data processing, to produce heterogeneous data. In prior art solutions, these data are currently stored in computer files having different and unrelated structures. This information is therefore quite difficult to archive, transfer and elaborate.

The genomic or proteomic sequences referred to in this invention include, for example, and not as a limitation, nucleotide sequences, Deoxyribonucleic acid (DNA) sequences, Ribonucleic acid (RNA), and amino acid sequences.

Sequence alignment refers to the process of arranging sequence reads by finding regions of similarity that may be a consequence of functional, structural, or evolutionary relationships among the sequences. When the alignment is performed with reference to a pre-existing nucleotides sequence referred to as "reference sequence", the process is called "mapping". Prior art solutions store such information in "SAM", "BAM" or "CRAM" files. The process of performing sequence alignment is also referred to as "aligning".

The concept of aligning sequences to reconstruct a partial or complete genome is depicted in figure 2 of WO2018068827 A1 whose disclosure is hereby incorporated by reference.

It exist a clear need to provide an appropriate genomic sequencing data and metadata representation (Genomic File Format) by organizing and partitioning the data so that the compression of data and metadata is maximized and several functionality such as selective access and support for incremental updates and other data handling functionality useful at the different stages of the genome data life cycle are efficiently enabled.

Moreover, when genome sequencing data generated by high throughput sequencing machines is analyzed by processing pipelines and analysts, annotations of different regions of the genome, expressing a number of diverse properties, are generated and currently represented by heterogeneous textual formats. Even though different types of generated results and annotations are conceptually related to each other and ideally need to be jointly accessed and used, the current solutions used in the art are such that these metadata are in the form of independent and separated text files and separated from the coded data related to the genomic reads. These formats do not support any type of linkage between the elements of one file with the elements of other files which are conceptually linked and thus may share a common biological meaning.

In the best case, such lack of explicit connection implies that processing and using genomic data and annotation information, requires time-consuming and overly inefficient parsing of possibly large text files when searching for specific information and associated metadata. In the worst case, the fact that it is not possible to describe connections, hampers the development of effective bioinformatics workflows and databases for downstream applications such as biomedical research or personalized medicine.

For example, RNA-sequencing reads, aligned onto a gene (which is typically composed by a set of intervals on a reference genome), need to be counted in order to measure the degree of expression of the gene in the biological condition used for the experiment. Different biological conditions (producing different sets of reads generated by different experiments) are usually compared in the context of specific experiment aiming at finding paths linking genotypes to phenotypes. The process of generating and aggregating information related to single reads and their alignments to a reference genome into results with a more general genetic and biological meaning, is referred to as "secondary analysis". Different types of annotations (meta-information) generated by secondary analysis using genome sequencing reads, can be conceptually associated to the genome sequencing reads aligned to one or more intervals of the genomic sequences used as references.

A genomic interval can be uniquely identified by specifying a sequence of nucleotides in the reference assembly (i.e. a chromosome in a genome, a gene, set of contiguous bases, a single base, ...), the molecule strand which can be forward or reverse, and a start and an end positions specifying the range of bases (a.k.a. nucleotides) included in the interval.

| **Interval** | | | |
|---|---|---|---|
| sequence identifier | strand | start position | end position |

Features associated with a genome interval such as variants, the number of aligned reads at a given position (also denoted as "coverage"), portions of the genome binding to proteins, nature and position of genes and regions associated to specific genetic functions can be uniquely identified and associated to genomic intervals. An interval can be as short as a single base, or it can span several thousand nucleotides or more.

A large number of integrated experiments can build a complex analysis of genome sequencing data. A different sequencing-derived protocol usually characterizes each experiment; it is used in order to sample a different function or compartment of the cell. The results produced by primary analysis (i.e. alignment of the reads with respect to a reference) and secondary analysis (i.e. integration and statistical studies performed on the results of the alignment) in each experiment can be visualized in graphical form using software applications called genome browsers, enabling one-dimensional navigation of the genome along the positions of nucleotides. The information resulting from secondary analysis associated to each position in the genome or to each interval is usually visualized in the form of different plots (or "tracks") per sequencing experiment, representing the presence and structure of transcripts, sequence variants in an individual or a population, coverage of sequencing reads, intensity of protein binding to each position of the genome.

State of the art genome annotation formats produced by analysis tools represent all the aforementioned results - also referred to as "features" - using a number of heterogeneous and independently defined and maintained formats. Such formats are usually characterized by poor and inconsistent syntaxes and semantics, which generate a proliferation of slightly different and incompatible file formats for each type of analysis result. The drawback of all the currently existing solutions is that the scientists working on integrative analysis of genomic data are forced to systematically transcode the different formats by using complex concatenations of text-processing tools and programs when sets of experiments need to be jointly accessed and studied. Such proliferation of different formats results in poor interoperability and reproducibility of results across different groups of scientists using even only slightly different representations and associated semantics.

The formats most used to represent genome annotations generated by genome sequencing data analysis and used in the art are:
- The Variant Calling Format (VCF) to represent variants with respect to a reference genome which can be present either in single individuals or populations of individuals;
- The Browser Extensible Data (BED) format which supports the representation of data lines that are displayed in an annotation track typically shown in genome browsers. http://genome.ucsc.edu/FAQ/FAQformat#format1
- The Generic Feature Format (GFF) represents genomic features in a text file characterized by 9 columns and tab-delimiters.
- The Gene Transfer Format (GTF) is an extension to, and backward compatible with, GFF.
- The BigWig format is used to represent dense, continuous data to be displayed in a genome browser as a graph.
- In addition, the fact that it is not possible to describe such heterogeneous data by means of a unified hierarchy implies that it is also utterly impossible to describe relations between features belonging to different categories, which makes advances in the field more difficult.

### SUMMARY

In order to solve the above problems of the existing prior art, the subject-matter of claims 1, 9, 12, 14 and 16 is proposed. Advantageous modifications are indicated in the dependent claims.

More specifically, the present disclosure provides a computer-implemented method for the encoding, storage and/or transmission of a representation of genome sequencing data in a genomic file format comprising annotation data associated with said genome sequencing data, said genome sequencing data comprising reads of sequences of nucleotides, said method comprising the steps of:
aligning said reads to one or more reference sequences thereby creating aligned reads,
classifying said aligned reads according to classification rules based on mapping of said aligned reads on said one or more reference sequences, thereby creating classes of aligned reads,
entropy encoding said classified aligned reads as a multiplicity of blocks of descriptors,
structuring said blocks of descriptors with header information thereby creating Access Units of a first sort containing genome sequencing data,
said method further comprising encoding annotation data into different Access Units of a second sort and indexing data into a master annotation index, wherein said indexing data represent an encoded form of annotation string data obtained by employing at least one compressed string indexing algorithm on said annotation string data, and wherein said MAI associates encoded annotation strings with said access units of a second sort.

Preferably, the method further comprises jointly coding said access units of first sort, of second sort and said MAI.

The method may further comprise a step of storing or transmitting the encoded genome sequencing data on or to a computer-readable storage medium; or making the encoded genome sequencing data available to a user in any other way known in the art, e.g. by transmitting the genome sequencing data over a data network or another data infrastructure.

In the context of this disclosure, descriptors may be implemented, e.g., as genomic annotation descriptors as defined in the detailed description below.

It is further preferable that said access units of the second sort containing genomic annotation data further comprise information data identifying a genomic interval, wherein said genomic interval identifies a sequence of nucleotides in the one or more reference sequences such that the annotation data contained in the access units of the second sort are associated with the related encoded reads of the genomic sequence contained in access units of the first sort containing genome sequencing data.

According to a (further) preferred embodiment, the encoding of said annotation data and indexing data comprises the steps of:
encoding genomic annotation data as genomic annotation descriptors, wherein said genomic annotation descriptors comprise numeric descriptors and textual descriptors, said encoding comprising the steps of:
- selecting a subset of textual descriptors from said textual descriptors according to a configuration parameter, in particular provided by the user;
- transforming said subset of textual descriptors by employing a first string transformation method to produce a string index;
- transforming and encoding said string index by employing a string indexing transformation method thereby producing master annotation index data;
- transforming said numeric descriptors and the textual descriptors not included in said subset of textual descriptors by employing at least one second transformation method different from the first transformation method;
- encoding said numeric descriptors and the textual descriptors not included in said subset of textual descriptors into separate access units of the second sort, by employing at least one first entropy encoder for the numeric descriptors and at least one second entropy encoder for the textual descriptors not included in said subset of textual descriptors.

It is further preferred that said first string transformation method comprises the steps of:
- inserting a string terminator character for signaling the termination of each textual descriptor, after each textual descriptor;
- concatenating the textual descriptors;
- interleaving genomic annotation record index data for associating said textual descriptors with the position of a genomic annotation record within the Access Unit of the second sort.

According to a (further) preferred embodiment, the string indexing transformation method is one of string pattern matching, suffix arrays, FM-indexes, hash tables.

Preferably, said at least one second transformation method is one of: differential coding, run-length coding, bytes separation, and entropy coders such as CABAC, Huffman Coding, arithmetic coding, range coding.

According to a (further) preferred embodiment, said master annotation index contains in its header the number of AU types and the number of indexes for each AU type.

Further preferably, the above-described method further comprises coding of classified unaligned reads.

The object of the invention is further solved by a method for the decoding and extraction of sequences of nucleotides and genomic annotations data encoded according to the method described above, said method comprising the steps of:
parsing a genomic data multiplex into genomic layers of syntax elements;
parsing compressed annotation data;
parsing a master annotation index;
expanding said genomic layers into classified reads of sequences of nucleotides;
selectively decoding said classified reads of sequences of nucleotides on one or more reference sequences so as to produce uncompressed reads of sequences of nucleotides;
selectively decoding said annotation data associated with said classified reads.

Preferably, said method further comprises decoding information data related to a genomic interval, wherein said genomic interval identifies a sequence of nucleotides in the one or more reference sequences such that the annotation data are associated with the related encoded reads of the genomic sequence.

It is further preferred that the method further comprises decoding the data encoded according to the method for the storage or transmission of a representation of genome sequencing data in a genomic file format comprising annotation data associated with said genome sequencing data described above.

According to a further aspect of the present disclosure, a genomic encoder for the compression of genome sequence data in a genomic file format comprising annotation data associated with said genome sequencing data is proposed, wherein said genome sequence data comprises reads of sequences of nucleotides, said and wherein said encoder comprises:
- an aligning unit for aligning said reads to one or more reference sequences thereby creating aligned reads;
- a data classification unit for classifying said aligned reads according to classification rules based on mapping of said aligned reads on said one or more reference sequences, thereby creating classes of aligned reads,
- entropy coding units for entropy encoding said classified aligned reads as a multiplicity of blocks of descriptors,
- an access unit coding unit for structuring said blocks of descriptors with header information thereby creating Access Units of a first sort containing genome sequencing data,
- a genomic annotation encoding unit for encoding annotation data into different Access Units of a second sort and indexing data into a master annotation index, wherein said indexing data represent an encoded form of annotation string data obtained by employing at least one compressed string indexing algorithm on said annotation string data, and wherein said MAI associates encoded annotation strings with said access units of a second sort.

Preferably, the encoder comprises means for jointly coding said access units of first sort, of second sort and said MAI.

According to a (further) preferred embodiment, the genomic encoder comprises encoding means for performing the steps of the encoding method described above.

The present disclosure further refers to a genomic decoder apparatus for the decoding of sequences of nucleotides and genomic annotations data encoded by the encoder described above, said decoder comprising:
- means for parsing a genomic data multiplex into genomic layers of syntax elements,;
- means for parsing said compressed annotation data;
- means for parsing a master annotation index;
- means for expanding said genomic layers into classified reads of sequences of nucleotides;
- means for selectively decoding said classified reads of sequences of nucleotides on one or more reference sequences so as to produce uncompressed reads of sequences of nucleotides;
- means for selectively decoding said annotation data associated to said classified reads.

Preferably, the genomic decoder further comprises decoding means for performing the steps of the decoding method described above.

According to a further aspect of the present disclosure, a computer-readable medium is proposed, the computer-readable medium comprising instructions that when executed by at least one processor, cause the at least one processor to perform the method described above.

### TERMINOLOGY

In this disclosure the following terms and expression are used:
**Bitstream syntax:** the structure of data coded as a sequence of bits (a.k.a. bitstream) in a digital data storage or communication application. The term refers to the format of a coded bitstream typically produced by an encoding application (a.k.a. encoder) and processed as input of a decoding application (a.k.a. decoder) to reconstruct the uncompressed data when compression is used. A bitstream syntax uses several syntax elements to represent the information coded in the bitstream.
**Syntax element:** component of the bitstream syntax representing one or more features of the coded information. In a bitstream generated by an encoder, syntax elements can be either compressed or not.
**Source model:** in information theory the expression "source model" designates the definition of the set of events generated by the source, their contexts and the probabilities associated to each event and corresponding context. In data compression, the knowledge of the source of the information to be coded is used to define a source model that makes possible to reduce the entropy of the model and as a consequence the number of bits needed to represent (i.e. code) the information generated by the source. **Sequencing data:** set of sequencing reads produced by a sequencing protocol.
**Sequencing read (a.k.a. read):** in sequencing, a read is an inferred sequence of base pairs (or base pair probabilities) corresponding to all or part of a nucleic acid molecule.
**Genomic interval:** succession of bases (a.k.a. nucleotides) comprised between a start position and an end position on a sequence of nucleotides such as for example a chromosome, a gene, a transcriptome or any other sequence of nucleotides.
**Genome feature:** set of genomic intervals sharing a biological property.
**Annotation data:** quantitative, qualitative or sequencing information associated with a genome feature. These include variants, browser tracks, functional annotations, methylation patterns and levels, sequencing coverage and statistics, feature expression matrices, contact matrices, affinity of a protein for nucleic acids.
**Functional annotation:** information associated with genomic features, in particular related to hierarchies of concepts related to the biological transcription and translation genomic information (gene, transcript, exon, coding sequences, etc.). Formats currently used to represent such information include GFF, GTF, BED and all their derivatives.
**Multiplexer:** coding module which receives as an input a multiplicity of Access Units of different types and generates a structured bitstream for streaming or file storage usage.
**Genomic annotation record:** data structure composed of a set of genomic annotation descriptors representing a genomic interval and annotation data related to genomic functional annotation, browsers tracks, genomic variants, gene expression information, contact matrices and other annotations associated to said genomic interval. One genomic annotation record can be logically linked to other genomic annotation records and the related annotations.
**String data structure:** data structure used to index strings allowing for fast searches, possibly in the compressed domain.
**Master Index Table (MIT):** indexing structure defined in ISO/IEC 23092-1 and WO2018068827A1 and WO2018152143A1. It is used to associate genomic intervals and classes of encoded genome sequencing reads with the Access Unit used to carry the compressed reads mapped on said intervals and associated metadata.
**Data Blocks, Access Units, Genomic Data Layer, Genomic Data Multiplex of genomic compression** The data structure further disclosed by this invention relies on the concepts of:
   A **Data Block** is defined as a set of the descriptor vector elements, of the same type (e.g. positions, distances, reverse complement flags, position and type of mismatch) composing a layer. One layer is typically composed by a multiplicity of data blocks. A data block can be partitioned into Genomic Data Packets described in co-pending patent application n. WO2018068830A1, herewith incorporated by reference, into which consist in transmission units having a size typically specified according to the communication channel requirements. Such partitioning feature is desirable for achieving transport efficiency using typical network communication protocols.
An **Access Unit** is defined as a subset of genomic data that can be fully decoded either independently from other access units by using only globally available data (e.g. decoder configuration) or by using information contained in other access units. An access unit is composed by a header and by the result of multiplexing data blocks of different layers. Several packets of the same type are encapsulated in a block and several blocks are multiplexed in one access unit. These concepts are depicted in Figure 5 and Figure 6 of WO2018068827. For clarity, in this disclosure, Access Units containing compressed genome sequencing data are referred to as Access Units of a first sort, whereas Access Units containing compressed annotation data are referred to as Access Units of a second sort.
A **Genomic Data Layer** is defined as a set of genomic data blocks encoding data of the same type (e.g. position blocks of reads perfectly matching on a reference genome are encoded in the same layer).
A Genomic Data Stream is a packetized version of a Genomic Data Layer where the encoded genomic data is carried as payload of Genomic Data Packets including additional service data in a header. See Figure 7 of WO2018068827 for an example of packetization of 3 Genomic Data Layers into 3 Genomic Data Stream.
A **Genomic Data Multiplex** is defined as a sequence of Genomic Access Units used to convey genomic data related to one or more processes of genomic sequencing, analysis or processing. Figure 7 of WO2018068827 provides a schematic of the relation among a Genomic Multiplex carrying three Genomic Data Streams decomposed in Access Units. The Access Units encapsulate Data Blocks belonging to the three streams and partitioned into Genomic Packets to be sent on a transmission network.

### BRIEF DESCRIPTION OF THE DRAWINGS

Figure 1 shows the relation between the present invention and the encoding apparatus described in ISO/IEC 23092.
Figure 2 shows an encoding apparatus for genomic annotations which works according to the principles of this invention and extends the encoding apparatus described in ISO/IEC 23092.
Figure 3 shows a decoding apparatus for genomic annotations which works according to the principles of this invention and extends the decoding apparatus described in ISO/IEC 23092.
Figure 4 shows a decoding apparatus for genomic annotations allowing partial decoding driven by textual queries which works according to the principles of this invention and extends the decoding apparatus described in ISO/IEC 23092.
Figure 5 shows an example of possible layout for the uncompressed index of a String Index, useful to illustrate the string indexing algorithm presented in this disclosure.
Figure 6 shows how to combine two families of string indexing algorithms in order to maximize compression and speed more than what would be possible by using only one family.

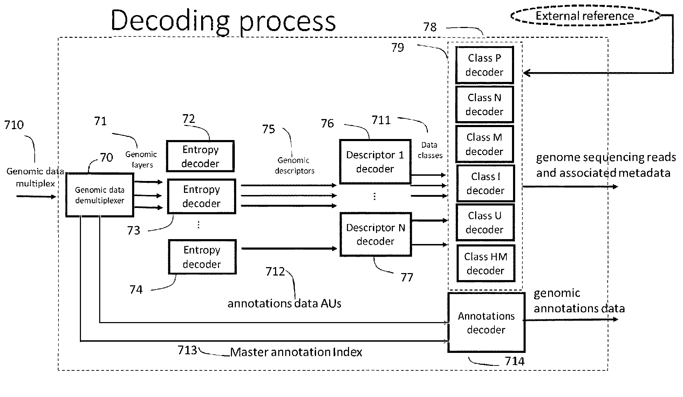

Figure 7 shows the relation between the present invention and the decoding apparatus described in ISO/IEC 23092.

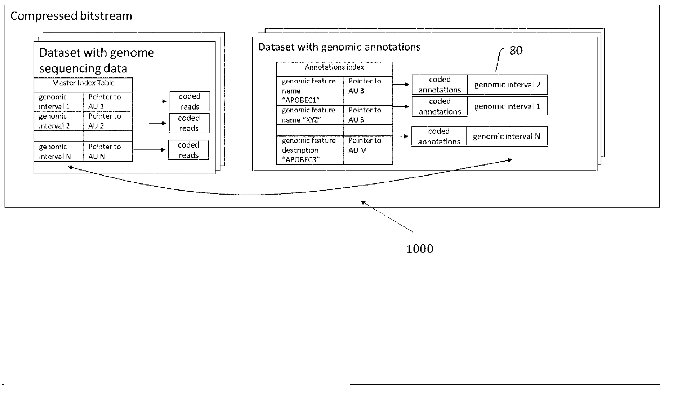

Figure 8 illustrates how the conceptual organization of data described in the present invention makes provision for textual queries to be performed.

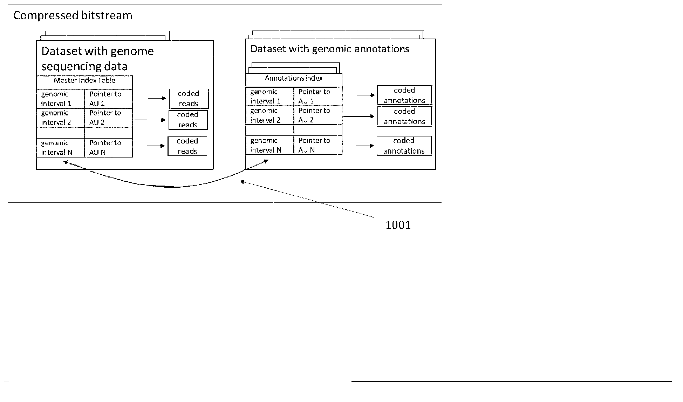

Figure 9 illustrates how the conceptual organization of data described in the present invention makes provision for searches over genomic intervals to be performed.

### DETAILED DESCRIPTION

Important aspects of the disclosed solution are:
1 The classification of the sequence reads in different classes according to the results of the alignment with respect to a reference sequence in order to enable selective access to encoded data according to criteria related to the alignment results. This implies a specification of a file format that "contains" structured data elements in compressed form. Such approach can be seen as opposite to prior art approaches, SAM and BAM for instance, in which data are structured in non-compressed form and then the entire file is compressed. A first clear advantage of the approach is to be able to efficiently and naturally provide various forms of selective access to the data elements in the compressed domain, which is impossible or extremely awkward in prior art approaches.
2 The decomposition of the classified reads into layers of homogeneous metadata in order to reduce the information entropy as much as possible. The decomposition of the genomic information into specific "layers" of homogeneous data and metadata presents the considerable advantage of enabling the definition of different models of the information sources characterized by low entropy. Such models not only can differ from layer to layer, but can also differ inside each layer. This structuring enables the use of the most appropriate specific compression for each class of data or metadata and portion of them with significant gains in coding efficiency versus prior art approaches.
3 The structuring the layers into Access Units, i.e. genomic information that can be decoded either independently by using only globally available parameteres (e.g. decoder configuration) or by using information contained in other Access Units. When the compressed data within layers are partitioned into Data Blocks included into Access Units different models of the information sources characterized by low entropy can be defined.
4 The information is structured so that any relevant subset of data used by genomic analysis applications is efficiently and selectively accessible by means of appropriate interfaces. These features enable faster access to data and yield a more efficient processing. A Master Index Table and Local Index Tables enable selective access to the information carried by the layers of encoded (i.e. compressed) data without the need to decode the entire volume of compressed data. Furthermore, an association mechanism among the various data layers is specified to enable the selective access of any possible combination of subsets of semantically associated data and/or metadata layers without the need to decode all the layers.
5 The joint storage of the Master Index Table and the Access Units.

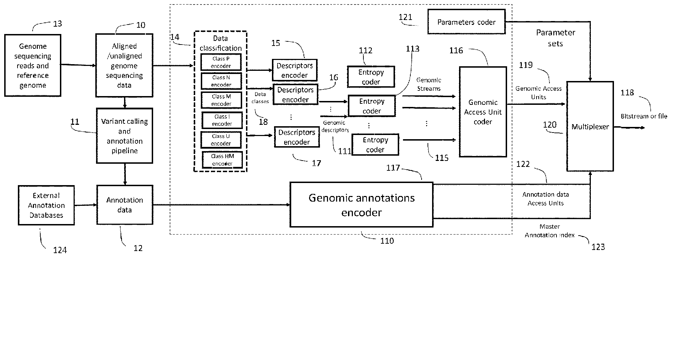

The encoding scheme of the genomic reads is represented in the encoder of figure 1.

### Classification of sequence reads

The sequence reads generated by sequencing machines are classified by the disclosed invention into five different "classes" according to the results of the alignment with respect to one or more reference sequences. Said classes are defined based on matching with/mapping on the reference genome according to the presence of substitutions, insertions, deletions and clipped bases with said one or more reference sequences.

When aligning a DNA sequence of nucleotides with respect to a reference sequence five are the possible results:
1. A region in the reference sequence is found to match the sequence read without any error (perfect mapping). Such sequence of nucleotides will be referenced to as "perfectly matching read" or denoted as "Class P".
2. A region in the reference sequence is found to match the sequence read with a number of mismatches constituted by a number of positions in which the sequencing machine was not able to call any base (or nucleotide). Such mismatches are denoted by an "N". Such sequences will be referenced to as "N mismatching reads" or "Class N".
3. A region in the reference sequence is found to match the sequence read with a number of mismatches constituted by a number of positions in which the sequencing machine was not able to call any base (or nucleotide) OR a different base than the one reported in the reference genome has been called. Such type of mismatch is called Single Nucleotide Variation (SNV) or Single Nucleotide Polymorphism (SNP). The sequence will be referenced to as "M mismatching reads" or "Class M".
4. A fourth class is constituted by sequencing reads presenting a mismatch type that includes the same mismatches of class M plus the presence of insertions or deletions (a.k.a. indels). Insertions are represented by a sequence of one or more nucleotides not present in the reference, but present in the read sequence. In literature when the inserted sequence is at the edges of the sequence it is referred to as "soft clipped" (i.e. the nucleotides are not matching the reference but are kept in the aligned reads contrarily to "hard clipped" nucleotides which are discarded). Deletion are "holes" (missing nucleotides) in the aligned read with respect to the reference. Such sequences will be referenced to as "I mismatching reads" or "Class I".
5. A fifth class includes all reads that do now find any valid mapping on the reference genome according to the specified alignment constraints. Such sequences are said to be Unmapped and belonging to "Class U".

Unmapped reads can be assembled into a single sequence using de-novo assembly algorithms. Once the new sequence has been created unmapped reads can be further mapped with respect to it and be classified in one of the four classes P, N, M and I.

Once the classification of reads is completed with the definition of the classes, further processing consists in defining a set of distinct syntax elements which represent the remaining information enabling the reconstruction of the DNA read sequence when represented as being mapped on a given reference sequence. A DNA segment referred to a given reference sequence can be fully expressed by:

### Syntax elements used in coding of genomic reads

- The starting position on the reference genome (pos).
- A flag signaling if the read has to be considered as a reverse complement versus the reference (rcomp).
- A distance to the mate pair in case of paired reads (pair).
- The value of the read length in case of the sequencing technology produces variable length reads. In case of constant reads length the read length associated to each reads can obviously be omitted and can be stored in the main file header.
- Additional flags describing specific characteristics of the read (duplicate read, first or second read in a pair etc... ).
- For each mismatch:
   ∘ Mismatch position (nmis for class N, snpp for class M, and indp for class I)
   ∘ Mismatch type (not present in class N, snpt in class M, indt in class I)
- Optional soft clipped nucleotides string when present (indc in class I).

This classification creates groups of descriptors (syntax elements) that can be used to univocally represent genome sequence reads.

For each layer of the genomic data structure disclosed in this invention different coding algorithms may be employed according to the specific features of the data or metadata carried by the layer and its statistical properties. The "coding algorithm" has to be intended as the association of a specific "source model" of the descriptor with a specific "entropy coder". The specific "source model" can be specified and selected to obtain the most efficient coding of the data in terms of minimization of the source entropy. The selection of the entropy coder can be driven by coding efficiency considerations and/or probability distribution features and associated implementation issues. Each selection of a specific coding algorithm will be referred to as "coding mode" applied to an entire "layer" or to all "data blocks" contained into an access unit. Each "source model" associated to a coding mode is characterized by:
- The definition of the syntax elements emitted by each source (e.g. reads position, reads pairing information, mismatches with respect to a reference sequence etc.)
- The definition of the associated probability model.
- The definition of the associated entropy coder.

For each data layer the source model adopted in one access unit is independent from the source model used by other access units for the same data layer. This enables each access unit to use the most efficient source model for each data layer in terms of minimization of the entropy

### Genomic annotations

Genomic annotations, browsers tracks, variant information, gene expression matrices and other annotations referred to in this invention are associated with, for example, and not as a limitation, nucleotide sequences, Deoxyribonucleic acid (DNA) sequences, Ribonucleic acid (RNA), and amino acid sequences. Although the description herein is in considerable detail with respect to annotations to a reference genome in the form of a nucleotide sequence, it will be understood that the methods and systems for compression can be implemented for annotations of other genomic or proteomic sequences as well, albeit with a few variations, as will be understood by a person skilled in the art.

Genomic functional annotations are defined as notes added by way of explanation or commentary to identified locations of genes and coding or non-coding regions in a genome to describe what is the function of those genes and their transcripts.

Genomic variants (or variations) describe the difference between a genomic sample and a reference genome. Variants are usually classified as small-scale (such as substitutions, insertions and deletions) and large-scale (a.k.a. structural variations) (such as copy number variations and chromosomal rearrangements).

Genome browser tracks are plots associated to aligned genome sequencing reads visualized in genome browsers. Each point in the plot corresponds to one position in the reference genome and expresses information associated to said position. Typical information represented as browser tracks is the presence and structure of transcripts, sequence variants in an individual or a population, coverage of sequencing reads, intensity of protein binding to each position of the genome, etc

Gene expression matrices are two-dimensional arrays where rows represent genomic features (usually genes or transcripts), columns represent various samples such as tissues or experimental conditions, and numbers counting the number of times each gene is expressed in the particular sample (the counter is also known as "expression level" of the particular gene).

Contact matrices are produced by Hi-C experiments and each i,j entry measures the intensity of the physical interaction between two genome regions i and j at the DNA level. At the lowest granularity, i and j denote two positions on the genome represented as a single sequence of all concatenated chromosomes.

### Limitations of current state of the art

To date, the classes of annotation data listed above are represented using different and incompatible textual formats usually compressed using general purpose text compressors such as gzip, bzip2 etc. In most cases, analysis programs process this information by first uncompressing the entire file and then parsing the decoded text to look for, and if present, to extract, the required piece of information. It is rather frequent for each of the formats used for each category of data to be independently, and sometimes drastically, modified by different users or groups of users to generate several "variations" or "dialects" of the same format. This fact generates serious interoperability problems and the need to "sanitize" each file format variation before being able to exchange data.

Another limitation of current formats is the lack of support for establishing links among different types of annotation data when represented in compressed form. For example, associating a set of variants to a given gene requires to:
1) decompress and parse a variant file (i.e. decompress a BCF into a VCF)
2) decompress and parse a gene annotation file (i.e. GTF/GFF)
3) establish the link using the genomic positions of respectively the variant and the gene as results of both parsing operations on the entire files, which would require another ad-hoc format that does not exist at the moment of this writing.

State of the art formats have the drawback of being stored on a different file. This is inefficient insofar as data compression is concerned, and does not support any efficient process to perform a query on a compressed file. Retrieving all variants related to a given gene XYZ and possibly at the same time the expression of that gene in a set of samples cannot be done without decompressing the whole concerned files and parsing all their content. The described process of associating variants to a gene today can only be achieved by combining several inefficient operations of data decompression, parsing and processing, and by describing relations between the different features by means of novel ad-hoc formats which are not currently available or standardized.

### Use Case: variant calling in a clinical setup

As an example, but not as a limitation, the method disclosed in this document addresses the drawbacks of current solutions when trying to determine variants of clinical relevance with a variant calling pipeline, and visualise the results in a way which allows clinicians to easily inspect and validate results. The goal is to use genome re-sequencing to identify variants which can be related to the manifestation of a disease or a particular phenotype of interest. Variants are determined by first aligning genome sequencing reads to a reference genome and subsequently using the alignment information at all positions, accumulated for all reads ("pileup"), to call genomic variants, such as Single Nucleotide Polymorphisms (SNPs), through a suitable variant calling program. Variant calling is a complex operation requiring complex pipelines of tools performing sophisticated processing. False positive or false negative results can arise due to a number of technical problems, such as fluctuations in coverage or the variant being located in a repetitive genome region. Due to these problems, in clinical setups the variants of potential clinical significance are usually validated manually by a human operator before being included in a medical report. However, data processing and validation requires the access and correlation of a number of information elements (genome sequence, genome annotation, reads alignment, sequencing coverage, sequencing pileup in the regions flanking the variant), each one typically stored in separated files and represented using a different file format. In particular, it is not possible with current technologies to explicitly state relations such as "this set of sequencing reads, aligned to this range of positions in the genome (i.e. interval), supports this variant, which is contained in this genomic feature" as the different entities (aligned reads, variants, genomic features) are represented in separated and different files. Today this result can only be achieved by:
1) Decompressing the various files to retrieve the original textual representation of the information for the entire sample.
2) Parsing the textual files searching for the feature of interest (e.g. genomic interval, gene name, annotation name etc.)
3) Possibly mapping (slightly) different names used in the different files to identify the same feature (different naming conventions exist to identify the same genomic features)
4) Aggregating the retrieved information in a single container and exposing it to the end user or the processing application in an application-specific format.

These various steps may require very long times up according to the sizes of the parsed textual files which can be in the range of several Gigabytes up to hundreds of Gigabytes.

The present invention aims at addressing these limitations by providing:
1. a unified compressed representation of annotations capable of representing the information content of: browsers tracks, genomic variants, gene expression data, contact matrices and other metadata associated to genome sequencing data
2. high compression performance of said unified representation resulting in higher compression ratios when compared to state of the art solutions
3. embedded indexing features providing explicit browsing capabilities of the annotations and metadata in the compressed domain. Said indexing features support the execution of sophisticated queries yielding hierarchies of related data structures containing biologically linked annotations, browsers tracks, genomic variants, gene expression information, contact matrices and other annotations associated to intervals of aligned genome sequencing data
4. mechanisms to explicitly link the indexed and compressed sequencing raw data and associated etadata with the indexed and compressed annotations. Such mechanisms enable the selective access in the compressed domain of annotations and the associated relevant sequence reads by querying either the compressed raw data or the compressed annotation data.

In this example of variant calling in a clinical setup, data processing and visualization are accomplished by encoding two distinct compressed data structures (that may or may not be contained in the same file) linked by a bidirectional indexing mechanism. Said data structure contain:
1. Genome sequencing reads and the related alignment information
2. Annotation information (annotations, browsers tracks, genomic variants, gene expression information, contact matrices and other annotations data) as described in the present disclosure.

In particular, the encoded information is contained in a hierarchical structure, as the one described in the present disclosure, linking:
1. Variants to their containing gene or genomic feature, if any, with details on the function and ontology of each gene
2. Variants to their supporting reads, i.e. to the reads supporting the variant being called
3. Each variant to the pileup profile obtained from the reads supporting the variant.
4. Any other kind of annotation information described previously.

Current state of the art technologies allow the representation of the different sources of information needed for genomic data annotation and variant calling separately (aligned reads with SAM/BAM/CRAM files, genome annotations with GTF/GFF3 files, variants with VCF/BCF files, plus various indexing file formats required to implement range searches). They do not support explicit representation of bidirectional relations between different entities. Moreover, a software analysis workflow (or "pipeline") performing variant calling needs to operate on different file formats depending on the analysis stage, rather than on a single data structure as provided by the present disclosure. It is possible to represent different sources of information as a single genome browser, but that requires the manipulation of a number of different file formats, and there is no way to specify to the genome browser that features belonging to different files are correlated.

### Technical advantage advantage for variant calling analysis.

In an embodiment, this invention presents important technical advantages for the use case of variant calling analysis as described in the text below.

The advantages of the present method with respect to state of the art solutions in terms of efficient data retrieval for variant calling analysis are the following.
1. Applications providing explicit representations of relations between sequencing reads and genomic features such as genome browsers have to support and manage a single data container and related bitstream format instead of a multiplicity of possibly non-interoperable formats.
2. By using genome browsers or other similar means, clinicians and scientists can explore the relation between variants, their supporting reads and the name and function of the containing gene or genes. In particular, the integration between the different types of information allows clinicians and scientists to validate the correctness of variant calling (for instance, excluding miscallings due to the presence of repetitive reads and/or repetitive reference regions, or to the lack of re-alignment whenever multiple indels are present at different positions; or checking the likely importance of the variant by the function of its containing gene, or its presence in a database of known variants).
3. Through the possibility of conducting textual searches of the meta-information contained in the files, clinician or scientists can correlate the presence/absence of multiple variants based on gene function (for instance by retrieving all variants contained in genes with similar functions or genes having multiple functional copies, or by retrieving all variants with similar clinical effect that are contained in known databases).
4. The analysis pipeline can operate with selective access on a single coded data structure throughout all stages (from alignment to variant calling), leading to a much simpler and economical software development/data access pattern and lower operational costs.
5. As relations are explicitly established when encoding the data, and all the relations are encoded in a browsable index rather than requiring decompression and parsing of entire and of possibly disconnected files, it is possible to discard irrelevant features (for instance, variants which are present in known databases, but not in the individual being re-sequenced, or variants which are irrelevant to the pathology being considered), thus obtaining higher compression.
6. All processing steps from 1 to 5 requiring data access, can be performed leveraging the indexing mechanisms embedded in the compressed data to support retrieval with a single query of both sequencing reads and all the associated annotations from a single compressed file structure. Said sequencing reads and the associated annotations can as well be decoupled and encapsulated in separate files to enable the transport of only the required portion of the data.

### Limitations of state of the art solutions for variant calling

State of the art technologies support the representation of the different pieces of information needed for the described use cases by using different data structures and formats (aligned reads with SAM/BAM/CRAM file formats, genome annotations with GTF/GFF3 file formats, variants with VCF/BCF file formats, plus various types of independent indexing file formats used to implement only range searches). These state of the art technologies do not support the explicit representation and linkage of relations between different pieces of information. A pipeline performing variant calling needs to operate on different file formats depending on the analysis stage, rather than on a single compressed data structure selectively accessible as proposed in the present approach. Employing current state of the art technology it is possible to feed a genome browser with the different pieces of genomic information, but this requires a complex pre-processing stage consisting of the manipulation and parsing of a number of different file formats in non-compressed form. Moreover, there is no way of specifying to the genome browser for appropriate display, the correlation between annotations, biological features and sequencing data.

### Use case: establishing and queriying a population-level library of genomic variants data

As an example, but not as a limitation, the method disclosed in this document addresses the drawbacks of existing solutions when trying to compile large databases of genomic variants. The scenario is similar to the one considered in the previous case, i.e a setup where researchers or clinicians are trying to validate and collect genomic variants based on sequecning techniques. However, we now assume that said researchers or clinicians are interested in cataloguing a large number of variants - ideally all the variants in each genome - for a potentially very large number of individuals (one could think about initiatives trying to cover an increasing portion of the population, with the final goal of covering it in its entirety). In this example, one would first perform variant callling and generally follow the analysis steps described in the previous use case; the process would then be repeated for all samples. After that, the researcher would usually query information about the results of data analysis, such as "How many individuals possess this specific variant?", or "Is this variant supported consistently in all the individuals considered?", or "How many people in the sample have any of the variants contained in a given dataset of clinically relevant variants? And what is the list of such variants for each individual?" At the moment, there are ways of storing the list of variables, typically as VCF/BCF files; however, the sizes of such population-level files are very large - which makes querying them technically challenging - and only very limited querying capabilities (i.e., retrieving variants in a specified genomic interval) are possible.

### Technical advantage

The advantages of the present method with respect to state-of-the-art solutions are the following ones:
1. The possibility of storing large collections of variants in a more compact way. That is due to the fact that the method disclosed in this document explicitly separates and describes the sources of information about the variants, thus making it possible to specify a better compression technique tailored to each information source
2. The possibility of performing more complex queries in the compressed domain. That is also due to the separation of data by individual and into several streams with a specified semantics, which make selective access and filtering possible in addition to range access based on genomic coordinates
3. The possibility of connecting information about variant calling with other kinds of information such as: the functional annotation present at the variant's position; the sequencing reads supporting each variant; the intensity at that position of some signal derived from other sequencing techniques, for instance from ChIP-seq experiments; etc.

### Limitations of state-of-the-art solutions

While storing large databases is possible by means of currently available formats such as VCF/BCF, the process is complex due to the complexity of the formats and the resulting files are relatively bulky due to the use of generic compression methods and because different sources of information are mixed together in the same record, making compression less efficient. In addition, formats such as VCF/BCF are not designed with complex queries in mind - it is only possible to query them by genomic range, in order to retrieve all the variants present in a genomic interval. Further filtering, such as selecting variants depending on whether they are present in some specified individual, must be performed separately. Finally, as described in the previous use case, there is no capability to cross information about genomic variants with other sources of information, such as lists of supporting sequencing reads or lists of functional genomic features.

### Use case: correlating information coming from complex omics experiments

As an example, but not as a limitation, the method disclosed in this document addresses the drawbacks and inefficiencies of current solutions when trying to determine biological mechanisms through which particular phenotypes originate. This is achieved by coding in the same compressed data structure several pieces of information (for instance, a number of "omics" sequencing-based experiments). The identification of complex molecular mechanisms requires the combination of a number of experimental techniques, each one probing a different cell compartment (for instance, ChIP-seq experiments investigating chromatin structure, bisulfite-sequencing experiments determining genome methylation, and RNA-seq experiments determining how transcription is regulated).

Molecular mechanisms underlying genotypes are determined by analysing the interaction and correlation between patterns occurring concurrently in different cell compartments when the same biological condition is sequenced. Chromatin markers are determined as peaks in ChIP-seq tracks, which are obtained by accumulating alignments to the reference genome; methylation patterns are obtained by special alignment pipelines able to process BS-seq data, as bisulfite treatment generates reads with modified bases whose sequence is not present in the original genome; RNA-sequencing data is processed by ad-hoc alignment pipelines able to perform spliced alignments, as the cell machinery derives RNA sequences by chaining together one or more blocks of genomic sequences ("exons") and discarding the sequences occurring between blocks ("introns"), which gives rise to sequences which are not present in the original genome; and so on, depending on the specific "omics" experiment being considered.

The data generated by each "omics" experiment usually requires complex analysis pipeline, each one tailored on the type of sequences being generated by the specific biological protocol employed (ChIP-seq, BS-seq, RNA-seq, etc.). Each pipeline usually requires a variety of types of data (genome sequence, genome annotation, sequencing reads, reads alignment, sequencing coverage, sequencing pileup), each one typically stored in a different file and represented using a different file format, to be considered and correlated. In particular, it is not possible with current technologies to explicitly state relations such as "in a given biological condition this set of sequencing reads, aligned to this range of positions in the genome, supports this ChIP-seq peak, which is correlated with particular patterns of RNA expression and genomic/histone methylation" as the different entities (aligned reads, ChIP-seq peaks, methylation patterns, genomic features, different biological conditions) are represented separately in different files.

### Technical advantage for data processing and visualization

In an embodiment, genomic data processing and visualisation are improved by means of the the present invention by presenting in the same compressed data structure:
1. Genome sequencing reads and the related alignment information
2. Annotation information (gene models, pileup profiles, methylation patterns, called ChIP-seq peaks, expression levels derived from RNA-sequencing) as described in the present disclosure.

In particular, the joint compressed data structure contains a hierarchical organization, as the one described in the present disclosure, linking:
1. Methylation patterns, ChIP-seq peaks and RNA expression, in different biological conditions, to their containing gene or genomic feature, if any, with details on the function and ontology of each gene
2. Methylation patterns, ChIP-seq peaks and RNA expression, in different biological conditions, to their supporting reads, i.e. to the reads supporting each feature being described
3. Each feature to the pileup profile obtained from the reads supporting the feature.

The advantages of the present method with respect to existing solutions in terms of efficient data retrieval for correlating information coming from several "omics" experiments are listed below.
1. As the present method provides explicit representation of relations between sequencing reads and "omics" features, and relations between different "omics" features, applications such as genome browsers have to support and manage a single data container and related bitstream format instead of a multiplicity of non-interoperable formats
2. Through the browser or other means, the researcher can explore the relation between the different "omics" features, their supporting reads and the name and function of the containing gene. In particular, the integration between the different types of information allows the researcher to infer correlations/causal relations between the different "omics" features highlighted by the experiment, flagging interesting genomic regions for subsequent experimental validation
3. Through the possibility of conducting textual searches of the annotations contained in the file, the researcher can correlate the presence/absence of multiple "omics" features based on gene function (for instance by retrieving all features contained in genes with similar functions or genes having multiple functional copies)
4. The analysis pipeline can operate on a single compressed data structure throughout all stages (from alignment to variant calling) and for all types of "omics" data, leading to a much simpler software development/data access pattern
5. As relations are explicitly established when encoding the file, and all the relations are encoded in the same file rather than using disconnected files, it is possible to discard irrelevant features (for instance, "omics" features that occur outside regions of interest), thus obtaining higher compression.

### Limitation of existing solutions for the linkage of different genomic features

Existing technologies allow users to represent the different sources of information needed for this use case separately (aligned reads with SAM/BAM/CRAM files, genome annotations with GTF/GFF3 files, ChIP-seq peaks, RNA expression levels and other "omics" feature with other file types, plus various indexing file formats required to implement range searches). They do not support the explicit representation of relations between different entities. A pipeline performing analysis of each kind of "omics" data needs to operate on different file formats depending on the analysis stage, rather than on a single compressed data structure as proposed in the present approach. It is possible to present different sources of information as a single genome browser, but that requires the manipulation of a number of different file formats, and there is no way of describing to the genome browser that features belonging to different files are correlated.

### Concepts and terminology

### Access Units

With reference to WO 2018/068827A1, WO/2018/068828A1 and WO/2018/068830A1 throughout this disclosure, an **Access Unit** (AU) is defined as a logical data structure containing a coded representation of genomic information to facilitate the bit stream access and manipulation. It is the smallest data organization that can be decoded by a decoding device implementing the invention described in this disclosure. An Access Unit is characterized by header information and a payload of compressed data structured as a sequence of blocks each one possibly compressed using different compression schemes.

The invention described in this document introduces new Access Units types containing genomic annotation data such as genomic features, functional annotations, browsers tracks, genomic variants, gene expression information, contact matrices, genotype data.

In th**e** context of this disclosure the following definitions apply:
**genomic annotation record:** data structure composed of a set of genomic annotation descriptors describing a genomic feature such as a genomic functional annotations, a browsers track, a genomic variant, gene expression information, contact matrices, genotype data and other annotations associated to genomic intervals. Each genomic annotation record is identified by a unique identifier as shown in T**a**bl**e** 1
**genomic feature**: a genomic feature is intended here as any piece of biologically meaningful information associated to genome sequencing data. As an example, but not as a limitation, genomic features include: g**eno**m**ic annotations**, b**ro**w**ser**s tracks, genomic variants, gene expression information, contact matrices. **access unit start position:** smallest mapping position on a reference sequence (for example a chromosome) for which the Access Units encodes genomic data or metadata.
**access unit end position:** largest mapping position on a reference sequence (for example a chromosome) for which the Access Units encodes genomic data or metadata..
**access unit range:** the genomic range comprised between the access unit start position and the access unit end position.
**access unit size:** number of genomic annotation records contained in an access unit.
**access unit covered region:** genomic range comprised between the Access Unit start position and the Access Unit end position.

In the context of this disclosure, one or more access units are organized in a structure called **genomic dataset.** A **genomic dataset** is a compression unit containing headers and access units. The set of access units composing the **genomic dataset** constitutes the **genomic dataset payload.**

A collection of one or more **genomic datasets** is called **dataset group.**

**read class:** ISO/IEC 23092 and WO 2018/068827A1, WO/2018/068828A1 and WO/2018/068830A1 and WO2018152143A1 specify how genome sequence reads are classified and encoded according to the result of the alignment of said reads on a reference genome. According to the type and number of mapping errors each read or read pair is assigned to a different class.

**AU class:** each AU contains reads belonging to a single class.

**Annotation data type:** in the context of this disclosure, annotation data types characterize the set of genomic annotation information included in one of these categories: genomic features, functional annotations, browsers tracks, genomic variants, gene expression information, contact matrices, genotype data, genomic samples information.

### Genomic annotation descriptors

In the context of this disclosure, **genomic annotation descriptors** are syntax elements representing part of the information (and also elements of a syntax structure of a file format and/or a bitstream) necessary to reconstruct (i.e. decode) coded reference sequences, sequence reads, associated mapping information, annotations, browsers tracks, genomic variants, gene expression information, contact matrices and other annotations associated to genome sequencing data. The genomic annotation descriptors which are common to all annotation data types disclosed in this invention are listed in Table 1.

Other descriptors specific to each annotation data type are disclosed in the syntax and semantics table devoted to each annotation data type.

Textual descriptors are those represented as string of characters while numeric descriptors are those represented by numerical values.

Genomic annotation descriptors can be of three types:
• Numeric descriptors represented as numerical values
• Textual descriptors represented as strings of characters
• Attributes are data structures defined in this disclosure (section titled "Attributes")

**Table 1 - Descriptors common to all annotation data types**

| **genomic annotation descriptor name** | **semantics** |
|---|---|
| ID | identifier of one genomic annotation record |
| parentID | identifier of a genomic annotation record linked to the one identified by ID by a "being parent" relation |
| pos | position of the coded annotation on the reference genome assembly used to generate said annotation |
| len | number of consecutive positions after the one identified by "pos" associated with the genomic annotation record identified by ID |
| strand | identifier of the genomic strand associated with the genomic annotation record identified by ID |
| name | textual name associated with the genomic annotation record identified by ID |
| description | textual description associated with the genomic annotation record identified by ID |
| attribute[] | one or more attributes associated with the genomic annotation record identified by ID. Attributes are structures as described in this disclosure |

According to the method disclosed in this invention, genomic annotations, browsers tracks, genomic variants, gene expression information, contact matrices and other annotation data types associated with genome sequencing data are coded using a sub-set of the descriptors listed in Table 1 which are then entropy coded using a multiplicity of entropy coders according to each descriptor specific statistical properties. This means that different types of descriptors are grouped together and coded with different entropy coders, thereby attaining higher compression. Blocks of compressed descriptors with homogeneous statistical properties are structured in Access Units which represent the smallest coded representation of one or more genomic feature that can be manipulated by a device implementing the invention described in this disclosure.

Genomic annotation descriptors are organized in **blocks** and **streams** as defined below.

A **block** is defined as a data unit composed by a header and a payload, which is composed by portions of compressed descriptors of the same type.

A **descriptor stream** is defined as a sequence of encoded descriptor blocks used to decode a descriptor of a specific Data Class.

This disclosure specifies a genomic information representation format in which the relevant information is efficiently compressed to be easily accessible, transportable, storable and browsable and for which the weight of any redundant information is reduced.

The main innovative aspects of the disclosed invention are the following.
1 Annotations, browsers tracks, genomic variants, gene expression information, contact matrices and other metadata associated to genome sequencing data are compressed in a unified hierarchical data structure. Said data structure enables fast transport, economical storage and the selective access to encoded data according to criteria such as by genomic interval/position, by gene name, by variant position and genotype, by variant identifier, by a comment in an annotation, by annotation type, by a pair of genomic intervals (in case of matrix data connecting genome positions to other positions).
2 The annotations, browsers tracks, genomic variants, gene expression information, contact matrices and other annotation data associated to genome sequencing data are represented by genomic annotation descriptors grouped in blocks with homogeneous statistical properties, enabling the identification of distinct information sources characterized by low information entropy.
3 The possibility of modeling each separated information source with distinct source models matching the statistical characteristics of each annotation descriptor and the possibility of changing the source model within each annotation descriptor for each annotation data type and within each descriptor block for each separately accessible data unit (Access Units). The adoption of the appropriate transformation, binarization and context adaptive probability models and associated entropy coders according to the statistical properties of each source model of annotation descriptors.
4 The definition of correspondences and dependencies among the descriptors blocks to enable the selective access to the sequencing data and associated metadata without the need to decode all the descriptors blocks if only part of the information is required.
5 The transmission of the configuration parameters governing the process of both encoding and decoding by means of data structures embedded in the compressed genomic data in the form of header information. Such configuration parameters can be updated during the encoding process in order to improve the compression performance. Such updates are conveyed in the compressed content in the form of updated configuration data structures.

In the following, each of the above aspects will be further described in detail.

### Genomic annotation descriptors per specific annotation data type

### Genomic variants

Data on genomic variants is encoded using the common descriptors introduced above and the specific descriptors listed below.

| **Descriptor** | **Type** | **Description** |
|---|---|---|
| ref_len | uint | |
| ref[ref_len] | c(1) | |
| alt_len | uint | |
| alt[alt_len] | c(1) | |
| filter[filter_len] | b(1) | bitmask on the list of filters present in the parameter set. |
| | | Filter_len is to be computed from the parameter set. |
| | | 1 value is reserved for MISSING, the other values refer to the list in the parameter set |
| qual_int | u(qual_int) | qual_int is defined in the parameter set |
| if (qual_type == 1) q_frac | u(qual_frac) | qual_frac is defined in the parameter set |
| info_mask[n_info] | b(1) | n_info is defined in the parameter set |
| info_values[n_info - info_null] | | info_null is the counter of 0 in info_mask |

### Functional annotations

Data on functional annotation describes genes and their content - spliced transcripts, with their biological function, in terms of their constituent exons; and information about the transcripts, such as, whenever applicable, their decomposition into UTRs, start and stop codon, and coding sequence. It is encoded using the common descriptors introduced above and the specific descriptors listed below.

| **Descriptor** | **Type** | **Description** |
|---|---|---|
| **type1** | uint | position in list defined in parameter set |
| **type2** | uint | position in list defined in parameter set |
| **phase** | uint | |
| **score** | f(32) | float 32 |
| **n_attributes** | uint | |
| for(a= 0; a < n_attributes; a++){ | | |
| **attr** | attribute | |
| **attr_value**[attr_size] | u(sizeof(attribute_type)) | |
| } | | |
| } | | |

sizeof() is a function which returns the number of bits necessary to represent each attribute value according to the type_ID defined in the attribute type.

### Tracks

Data for a track represents a numerical value associated to each position in the genome - a typical example for it would be the coverage of sequencing reads at each position as produced by an RNA- or ChIP-sequencing experiment. Data can be provided at different pre-computed zooming level, which is desirable when the information is being displayed in a genome browser. Data is encoded using the common descriptors introduced above and the specific descriptors listed below.

| **Descriptor** | **Type** | **Description** |
|---|---|---|
| **for(i=0; i < zoom_levels; i++)** | | zoom_levels and zoom_span as defined in the parameter set |
| **if(zoom_span[i] == 0)** | | |
| **values[1]** | | |
| **else** | | |
| **values[Ceil(len/zoom_span[i])]** | | |

### Genotype information

Genotype information data expresses the set of genomic variants present at each position of the genome for an individual or a population of individuals. It is encoded using the common descriptors introduced above and the specific descriptors listed below.

| **Descriptor** | **Type** | **Description** |
|---|---|---|
| **sample_id_start** | uint | |
| **sample_id_len** | uint | |
| **format_mask**[n_format] | b(1) | n_format is defined in the parameter set |
| if(format_ID[0] == 0){ | | format_ID is defined in the parameter set this signals the presence of genotyping information in the AU. It is signaled in the Parameter Set |
| genotype_present = 1 | | |
| **first_allele** | u(ceil(log2(alt_len +1))) | alt_len is specified in the parameter set |
| for(i=0; i < ploidy - 1; i++) { | | |
| **phase** | b(1) | |
| **allele** | u(ceil(log2(alt_len +1))) | |
| } | | |
| } | | |
| else genotype_present = 0 | | |
| for(i = genotype_present; i < n_format; i++){ | | |
| if(format_mask[i]) | | |
| **format_value**[value_len] | u(8) | value_len shall be inferred from the parameter set for each format specifier |
| } | | |

### Sample information

Information on samples describes meta-information on specific biological samples on which the sequencing experiment has been conducted, such as collection date and location, sequencing date, etc. Sample information data is encoded using the specific descriptors listed below.

| **Descriptor** | **Type** | **Description** |
|---|---|---|
| **sample_name** | st(v) | |
| **UUID** | uint | Unique identifier used to link with a Dataset in part 1 |
| **bitmask** | b(n_meta) | |
| **values[n_meta]** | uint | **n_meta is in the parameter set** |
| **desc_len** | uint | |
| **description** | u(desc_len) | |
| **n_attributes** | | |
| **attributes[n_attributes]** | attribute | e.g. URL of DOI to publication |

### Expression Information

Information on expression associates some genomic range (typically corresponding to a gene, a transcript or another feature in the genome) with one or more numerical values - each value would correspond to a biological condition that has been tested during a separate experiment. Expression data is encoded using the specific descriptors listed below.

| **Syntax** | **Type** | **Description** |
|---|---|---|
| **ID** | uint | Scope: AU |
| **feature_position** | uint | position of the feature in the parameter set list |
| **sample_id_start** | uint | |
| **sample_id_len** | uint | |
| **format_mask**[n_format] | b(1) | n_format is defined in the parameter set |

### Contact matrices information

Contact information data is encoded using the specific descriptors listed below.

| **Syntax** | **Type** | **Description** |
|---|---|---|
| **ID** | uint | Scope: AU |
| **start_position_x** | uint | Start position of the interval to which the coded values are referring |
| **length_x** | | Length of the interval to which the coded values are referring |
| **start_position_y** | uint | Start position of the interval to which the coded values are referring |
| **length_y** | | Length of the interval to which the coded values are referring |
| **format_mask**[n_format] | b(1) | n_format is defined in the parameter set |
| for(i = 0; i < n_format; i++){ | | |
| if(format_mask[i]) | | |
| for(i=0; i < zoom_levels; i++) | | zoom_levels and zoom_span as defined in the parameter set |
| if(zoom_span[i] == 0) | | |
| values[1] | | |
| else | | |
| values[Ceil(value_len/zoom_span[i])] | | value_len shall be inferred from the parameter set for each format specifier |
| } | | |

### Bitstream structure

The present invention introduces a compressed representation of annotation data associated with genome sequencing data in the form of a bitstream syntax described below. The syntax is described in terms of the concatenation of data structures composed by elements characterized by a data type.

### Syntax notation

In the following description the following syntax notation is adopted.

| | |
|---|---|
| uint | unsigned integer |
| int | signed integer |
| u(n) | unsigned integer represented with n bits |
| s(v) | variable length string |
| c(n) | n characters |
| f | fractional number |
| b(n) | n bits |
| comp_index(size) | data compressed using a compressed full-text substring index based for example, but not as a limitation on the Burrows-Wheeler transform such as the fm-index. |
| | "size" is the size in bytes of the compressed output |
| gen_info | Data structure of type gen_info as defined in ISO/IEC 23092-1 |

### Extension of ISO/IEC 23092-1

The present disclosure extends the data structures specified in ISO/IEC 23092-1 in order to support the transport of coded genomic annotation in the bitstream syntax specified in ISO/IEC 23092-1.

### Dataset group

The dataset group syntax is the same as the one specified in ISO/IEC 23092-1

| **Syntax** | **Key** | **Type** |
|---|---|---|
| *dataset_group {* | *dgcn* | |
| dataset_group_header | dghd | gen_info |
| reference[] | rfgn | gen_info |
| reference_metadata[] | rfmd | gen_info |
| label_list | labl | gen_info |
| DG_metadata | dgmd | gen_info |
| DG_protection | dgpr | gen_info |
| for (i=0;i<num_datasets;i++) { | | |
| dataset[i] | dtcn | gen_info |
| } | | |
| *}* | | |

### Dataset

In ISO/IEC 23092-1 a dataset is a data structure containing a header, Master configuration parameters in a parameter set an indexing structure and a collection of access units encoding genomic data. Dataset types are extended to carry genomic annotation data of different types specified by different "dataset_type" values.

| **Syntax** | **Key** | **Type** |
|---|---|---|
| *dataset {* | *dtcn* | |
| dataset_header | dthd | gen_info |
| DT_metadata | dtmd | gen_info |
| DT_protection | dtpr | gen_info |
| dataset_parameter_set[] | pars | gen_info |
| if (MIT_flag){ | | |
| master_index_table | mitb | gen_info |
| } | | |
| if(dataset_type > 2 && dataset_type < 8){ | | |
| master_annotation_index | maix | gen_info |
| if(dataset_type == DS_GENOTYPE ∥ dataset_type == DS_EXPRESSION){ | | |
| DT_annotation_meta_data | dtam | |
| } | | |
| } | | |
| access_unit[] | aucn | gen_info |
| if (block_header_flag == 0) { | | |
| descriptor_stream[] | dscn | gen_info |
| } | | |
| } | | |

| **dataset_type value** | **value name** | **Semantics** |
|---|---|---|
| 0 | DS_NON_ALIGNED | dataset containing non aligned content |
| 1 | DS_ALIGNED | dataset containing aligned reads |
| 2 | DS_REFERENCE | dataset containing a reference |
| 3 | DS_INTERVALS | dataset containing information related to a genomic interval |
| 4 | DS_GENOTYPE | dataset containing genotyping information |
| 5 | DS_EXPRESSION | dataset containing expression information |
| 6 | DS_CONTACTS | dataset containing contacts matrices |
| 7 | DS_STATISTICS | dataset containing statistics |

| **reference_type value** | **value name** | **Semantics** |
|---|---|---|
| 0 | MPEGG_REF | reference sequence |
| 1 | MPEGG_ANNOTATION_REF | reference data used for annotations |

### Dataset Header

This is a box describing the content of a dataset.

| **Syntax** | **Key** | **Type** |
|---|---|---|
| *dataset_header {* | *dthd* | |
| dataset_group_ID | | uint |
| dataset_ID | | uint |
| version | | c(4) |
| multiple_alignment_flag | | uint |
| byte_offset_size_flag | | uint |
| non_overlapping_AU_range_flag | | uint |
| pos_40_bits_flag | | uint |
| block_header_flag | | uint |
| if (block_header_flag) { | | |
| MIT_flag | | uint |
| CC_mode_flag | | uint |
| } | | |
| else { | | |
| ordered_blocks_flag | | uint |
| } | | |
| seq_count | | uint |
| if (seq_count > 0) { | | |
| reference_ID | | uint |
| for (seq=0;seq<seq_count;seq++) { | | |
| seq_ID[seq] | | uint |
| } | | |
| for (seq=0;seq<seq_count;seq++) { | | |
| seq_blocks[seq] | | uint |
| } | | |
| } | | |
| dataset_type | | uint |
| if (MIT_flag == 1) { | | |
| num_classes | | uint |
| for (ci=0 ;ci<num_classes ;ci++) { | | |
| clid[ci] | | uint |
| if (block_header_flag) { | | |
| num_descriptors[ci] | | uint |
| for(di=0;di<num_descriptors[ci];di++) { | | |
| descriptor_ID[ci] [di] | | uint |
| } | | |
| } | | |
| } | | |
| } | | |
| alphabet_ID | | uint |
| if(dataset_type < DS_INTERVAL){ | | |
| num_U_access_units | | uint |
| if (num_U_access_units > 0) { | | |
| reserved | | uint |
| U_signature_flag | | uint |
| if(U_signature_flag) { | | |
| U_signature_constant_length | | uint |
| if [U_signature_constant_length){ | | |
| U_signature_length | | uint |
| } | | |
| } | | |
| } | | |
| } | | |
| if (seq_count > 0) { | | |
| tflag[0] | | uint |
| thres[0] | | uint |
| for (i=1;i<seq_count;i++) { | | |
| tflag[i] | | uint |
| if(tflag[i] == 1) | | |
| thres[i] | | uint |
| else /* tflag[i] == 0 */ | | |
| /* thres[i] = thres[i-1] */ | | |
| } | | |
| } | | |
| while( !byte_aligned() ) | | |
| nesting_zero_bit | | uint |
| *}* | | |

### Reference

This data structure extends the reference data structure specified in ISO/IEC 23092 to support the bitstream syntax specified in this disclosure.

| **Syntax** | **Key** | **Type** |
|---|---|---|
| *reference {* | *rfgn* | |
| dataset_group_ID | | uint |
| reference_ID | | uint |
| reference_name | | st(v) |
| reference_major_version | | uint |
| reference_minor_version | | uint |
| reference_patch_version | | uint |
| seq_count | | uint |
| for (seqID=0;seqID<seq_count;seqID++) { | | |
| sequence_name[seqID] | | st(v) |
| if (minor_version != '1900') { | | |
| sequence_ID | | uint |
| ref_seq_checksum[seqID] | | uint |
| } | | |
| } | | |
| reserved | | uint |
| external_ref_flag | | uint |
| if (external_ref_flag) { | | |
| ref_uri | | st(v) |
| checksum_alg | | uint |
| reference_type | | uint |
| if (reference_type == MPEGG_REF ∥ reference_type == MPEGG_ANNOTATION_REF) { | | |
| { | | |
| external_dataset_group_ID | | uint |
| external_dataset_ID | | uint |
| if (minor_version == '1900') | | |
| ref_checksum | | u(checksum_size) |
| } | | |
| else if (minor_version == '1900') { | | |
| for(seqID=0;seqID<seq_count;seqID++) { | | |
| ref_seq_checksum[seqID] | | u(checksum_size) |
| } | | |
| } | | |
| } | | |
| else { | | |
| internal_dataset_group_ID | | uint |
| internal_dataset_ID | | uint |
| } | | |
| } | | |

### Annotation Indexing

The present disclosure describes how to encode (i.e compress) the annotation data portion composed of textual information elements associated with genome sequencing reads, other non textual genomic annotations and sequences derived from the genome so as to make the textual elements searchable in the compressed domain. Examples include:
- Information on functional genomic features (e.g. gene name, gene description, gene annotation, gene ontology, variant name, variant description, variant clinical significance)
- Nucleic acid sequences (such as subsequences of the reference genome, sequences of RNA molecules transcribed from the reference genome, or sequencing reads from the genome) represented as a sequence of symbols, typically one for each nucleotide
- Protein sequences (such as the sequences corresponding to the translation of messenger RNA molecules) represented as a sequence of symbols, typically one for each amino acid
- Information about sample meta-data and methodology (names, collection date/time/place, experimental techniques used to perform sequencing, analisys techniques used to perform functional annotation and variant calling, etc.).

Said information is compressed using a suitable data structure, such as, as an example and not as limitation, compressed string pattern matching data structures. Representatives of compressed string pattern matching data structures are, as examples and not as limitations, compressed suffix arrays, FM-indexes, and some categories of hash tables. Such (compressed) data structures are used to perform string pattern matching, and to carry in compressed form the textual portion of the annotational data being added to the compressed bitstream either in the file header or as a payload of an Access Unit. For clarity, in this disclosure all algorithms belonging to one of these data structure categories will be referred to as "string indexing algorithm".

As an example, but not as a limitation, this disclosure describes how to encode the textual portion of the different annotation data types and the genomic reads by using a combination of compressed string indexing algorithms. Several families of string indexing algorithms exist, and each family can be parameterized by a number of parameters, which specify the balance between compression performance and querying speed. We use for compression a pre-determined set of compressed string indexing algorithms, each one specified by the choice of a compressed string indexing algorithm family and by a choice of parameters for that family. The set of algorithms is sorted by the compression level attained, and, depending on the desired trade-off between compression rate/querying speed, one specific algorithm can be selected when encoding. This choice is specified in the parameter set of the compresssed bitstream.

As an example, but not as a limitation, the chosen compressed string indexing algorithm is separately or jointly applied to the concatenation of:
- gene name,
- gene description,
- sequences of genomic transcripts and their protein products, if any
- variant name,
- variant description,
- samples name,
- genome sequencing reads represented as a sequence of symbols one for each nucleotide and any other textual information associated with a genomic interval
- additional information encoding the relation of the textual information with genomic intervals.

Applying a compressed string indexing algorithm to said information produces a compressed and indexed representation which can be queried for the presence of arbitrary substrings. In particular, a combination of exact substring searches can be used to perform inexact substring searches, for example searches that retrieve all occurrences of substrings with up to a specified number of deviations (mismatches/errors) from the specified pattern. This process enables querying for a piece of textual information the genomic annotations considered or produced during analysis and re-analysis of sequencing data, in a single query. This is possible if:
1. Genomic information associated with a genomic interval is represented as a data structure called Genomic annotation record, which contains information related to the sequence of nucleotides included in said interval
2. Genomic annotation records associated with genomic intervals which are related to contiguous positions on the reference genome are compressed in the same Access Unit
3. All textual portion of the annotation information is compressed using a compressed string indexing algorithm chosen from the available set.

The following text and data structures describe an embodiment of this method for the indexing and search of genomic annotation data compressed and embedded in access units of a bitstreams compliant with MPEG-G (ISO/IEC 23092).

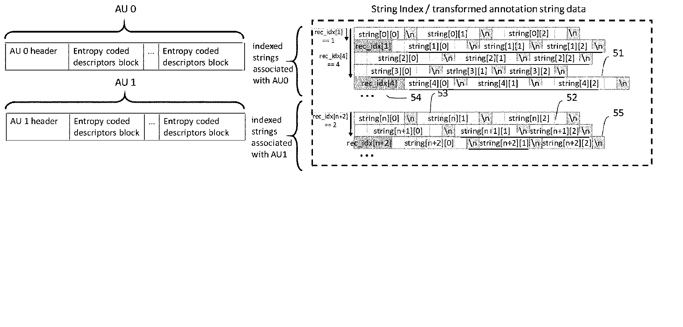

The table below shows the textual information indexed and compressed using a string indexing algorithm per each genomic annotation type according to the method described in this document. For each Access Unit, textual descriptors of each type are concatenated using a string separator and record indexing information as shown in figure 5 and compressed using a string indexing algorithm.

| **Data type** | **Strings per record** | **Description** |
|---|---|---|
| Variants, functional annotation, tracks, expression matrices, genotype information, contact information, samples information | Name, Description, other textual descrptors specific to each data type | Indexed textual descriptors are concatenated and encoded using the compressed string indexing algorithm of choice |

### Indexing criteria per genomic annotation Access Unite type

This table describes the indexing criteria and indexing tools applied to Access Units for each genomic annotation data type.

| **AU type ID** | **AU type alias** | **indexing criteria** | **indexing tool** |
|---|---|---|---|
| 0 | AU_TRACKS | seqID, start, end | MIT on genomic intervals |
| 1 | AU_VARIANTS | seqID, start, end | MIT on genomic intervals |
| | | variant | Variant name and description (MAI) |
| 2 | AU_FUNCTIONAL_ANN OTATIONS | seqID, start, end, | MIT on genomic intervals |
| | | feature | each feature is associated to a name and a ID which corresponds to its position in the ordered list present in the parameter set |
| | | | name and description (MAI) |
| 3 | AU_GENOTYPE | seqID, start, end, | sample_ID intervals |
| | | variant | each variant can be searched by name |
| | | Sample | each sample is associated with a name and a ID which corresponds to its position in the ordered list present in the parameter set |
| 3 | AU_EXPRESSIONS | seqID, feature, sample_ID | sample_ID intervals |
| | | | feature_ID intervals |
| | | | each feature and sample is associated with a name and a ID which corresponds to its position in the ordered list present in the parameter set |
| 4 | AU_CONTACTS | seqID, start, end | MIT on genomic intervals |
| 5 | AU_SAMPLES | sample_ID | sample_ID intervals |
| | | | each sample is associated with a name and a ID which corresponds to its position in the ordered list present in the parameter set |
| 6 | AU_STATS | seqID, start, end | MIT on genomic intervals |

The Master Annotation Index (MAI) is an indexing tool which provides for annotation data the indexing capabilities of sequence reads of the MIT defined in ISO/IEC 23092-1 and WO 2018/068827A1, WO/2018/068828A1 and WO/2018/068830A1

**Table 2 - Master Annotation Index**

| **Syntax** | **Key** | **Type** | **Remarks** |
|---|---|---|---|
| master_annotation_index { | *maix* | | |
| master_annotation_index_header () | *mahd* | | |
| for(i = 0; | | | num_mai_AU_types as specified in this disclosure |
| i < num_mai_AU_types; | | | |
| i++) { | | | |
| for(j = 0; | | | num_mai_indexes[]as specified in this disclosure |
| j < num_mai_indexes[i]; | | | |
| j++) { | | | |
| annotation_index[i][j] | *aidx* | *strin g_ind ex ()* | string_index() encoding a list of strings using a compressed string indexing algorithm as specified in this disclosure |
| } | | | |
| } | | | |
| } | | | |

### Master Annotation Index Header

**Table 3 - Master Annotation Index Header**

| **Syntax** | **Key** | **Type** | **Remarks** |
|---|---|---|---|
| master_annotation_index_header { | *mahd* | | |
| **reserved** | | uint | |
| **num_mai_AU_types** | | uint | Number of AU types indexed by MAI |
| for(i = 0; | | | |
| i < num_mai_AU_types; | | | |
| i++) { | | | |
| **reserved** | | uint | |
| **mai_AU_type[i]** | | uint | i-th AU type indexed by the MAI |
| **num_mai_indexes[i]** | | uint | Number of MAI indexes for the AU type mai_AU_type]i] |
| } | | | |
| } | | | |

### Semantics

**num_mai_AU_types** is the number of AU types indexed by MAI. A value of 0 signals that no indexing is provided by the MAI.
**mai_AU_type[i]** is the i-th AU type indexed by the MAI. The array mai_AU_type[] shall contain unique values, that is each AU type value can appear only once in the array mai_dataset_ID[].
**num_mai_indexes[i]** is the number of MAI indexes for the AU type mai_AU_type[i].

### Indexed Strings

When encoding an Access Unit of each genomic annotation data type, textual descriptors belonging to data encoded in said access unit are concatenated and compressed using a compressed string indexing algorithm as defined in this disclosure.

The table below lists which strings are encoded in a MAI, for each data type. The specified list of strings determines the value numStrings that is required in some of the following description for MAI. numStrings is the number of textual fields per genomic annotation record indexed using the method described in this invention.

| **Data type** | **Strings per record** | **Description** |
|---|---|---|
| Variants, functional annotation, tracks, expression matrices, genotype information, contact information, samples information | Name, Description, other textual descrptors specific to each data type | Indexed textual descriptors are concatenated and encoded using the compressed string indexing algorithm of choice |

### String Index

A String Index block is a portion of a Master Annotation Index that encodes one or more strings for each Record, for a variable number of Access Units each containing a variable number of Records.

The Master String Index also allows string pattern matching queries on the original text to be performed and retrieved.

The list of strings encoded within a String Index is referred to in the following as "compressed index".

The list of strings obtained by decoding a compressed index from a String Index is referred to in the following as "uncompressed index".

The String Index provides the following functionalities:
1. Count the occurrences of any substring within the list of encoded strings, as specified in the description below.
2. For each of the substrings found at previous point 1, retrieve the position of the substring within the uncompressed index, as specified in the description below.
3. Given a start and end position within the uncompressed index, retrieve the corresponding decoded payload, as specified in the description below, where the said payload may contain any number of strings, or of portions of strings, or of metadata associated to the strings.
4. For each of the substrings found at previous point 1, retrieve the whole string that contains the said substring, as well as the position of the said whole string within the uncompressed index, as specified in the description below.
5. For each of the substrings found at previous point 1, retrieve the index of the Access Unit within which the said substring is contained, as specified in the description below.
6. For each of the substrings found at previous point 1, retrieve the Record index of the Record within which the said substring is contained, where the said Record index is the 0-based index of the said Record within the Access Units that contains the said Record, as specified in the description below.
7. Given an Access Unit index, retrieve the position within the uncompressed index of the first string within the Access Unit corresponding to said Access Unit index, as specified in the description below.
8. Given an Access Unit index, a Record index within the Access Unit corresponding to said Access Unit index, and a string index within the Record corresponding to said Record index, retrieve the position of the string at the said string index contained in the said Record of the said Access Unit, as specified in the description below.

Inputs to this process are:
- a variable numAUs that specifies the number of Access Units for which strings are encoded within this String Index
- a variable codingMode that specifies the algorithm which has been used to encode the string index.

The number of strings encoded for each record shall be the same for all records, and it shall correspond to the variable numStrings as specified in the description below.

**Table 4 - String Index block.**

| **Syntax** | **Key** | **Type** | **Remarks** |
|---|---|---|---|
| string_index { | *msix* | | |
| **num_AUs** | | uint | Number of Access Units encoding in this String Index |
| for (i = 0; i < num_AUs; i++) { | | | |
| **au_id[i]** | | uint | Access Unit ID of the i-th Access Unit encoded in this String Index |
| if (i > 0) { | | | |
| **au_offset[i]** | | uint | Byte position in the uncompressed index corresponding to **compressed_index** of the first string of the first record of the i-th Access encoded in this String Index. |
| } | | | |
| } | | | |
| **coding_mode** | | uint | MAI coding mode. A parameter selecting the possible indexing configurations. |
| **reserved** | | uint | |
| **size** | | uint | Size in bytes of **compressed_index** element. |
| **compressed_index** | | uint [size] | a compressed list of strings using a compressed string indexing algorithm as specified in the description below |
| } | | | |

The uncompressed index encoded within **compressed_index** contains a list of strings and the associated optional record indexes, ordered per Access Unit (following the same order of the Access Units in Table 4) and, for each Access Unit, per Record (following the same order of the Records within the Access Unit). The total number of strings in the uncompressed index is totNumRecords*numStr**i**ngs, where totNumRecords is the total number of records of all Access Units identified by **au_id[]**, and numStrings is a counter of all strings compressed using said compressed indexing algorithm.

The uncompressed index specified as:

**Table 5 - Uncompressed index encoded in the compressed_index element of an string_index() element.**

| **Element** | **Type** | **Comment** |
|---|---|---|
| uncompressed_index(si) { | | si is a String Index block. uncompressed_index(si) is the result of decoding si.compressed_index. |
| for(i = 0; i < totNumRecords; i++) { | | totNumRecords is the total number of records of all Access Units identified by si.au_id[] |
| **record_index[i] []** | uint [n] | Genomic annotation record index data: with n comprised between 0 (i.e. element is not present) and 5. |
| | | All bytes in **record_index[i] []** shall have the most significant bit (i.e. value 0x80) set. |
| | | If n > 1, **record_index[i] [0]** shall not be equal to 0x80. |
| for(j = 0; j < numStrings; j++) { | | numStrings is the number of indexed strings |
| **string[i] [j] []** | uint [n] | Variable-size string. All bytes in **string[i] [j] []** shall be in the [0x20 .. 0x7f] range. |
| **string_terminator** | uint | String terminator, equal to value 0x0A (i.e. '\n') |
| } | | |
| } | | |
| } | | |

An example, with numStrings equal to 3, of the uncompressed index specified in this disclosure is provided in Figure 5.

### Semantics

**record_index[i]** (rec_idx), whose presence is signaled by setting the most significant bit on all the bytes of **record_index[i].** Setting the most significant bit also prevents from obtaining false-positive results when searching for sub-strings, since all bytes in **string[i][j]** field have the most significant bit unset as specified in this disclosure for **string[i][j]** element.

When **record_index[i]** is present and it is N bytes long, it represents a non-negative integer value as specified in the following expression: $recordIndexValue \left[i\right] = {\sum }_{n = 0}^{N - 1} \left(record_index\left[i\right]\left[n\right]&0x7F\right) ≪ \left(\left(N-1-n\right)∗7\right)$ where recordIndexValue[i] corresponds to the 0-based index, within the corresponding Access Unit, of the Record corresponding to **string[i][]** strings.

In the context of this disclosure, **record_index[i]** is referred to as "genomic annotation record index data".
**string[i][j]** is the j^{th} encoded string of the i^{th} record. The strings shall be ordered per Access Unit (following the same order of the Access Units in Table 4) and, for each Access Unit, per Record (following the same order of the Records within the Access Unit)
**string_terminator** is a single byte equal to 0x0A (i.e. '\n').

### Searching for substring positions with the String Index

The positions within the uncompressed index of a given substring are searched with String Index as specified in the following pseudocode:

**Table 6 - Searching substring positions with the String Index.**

| **Pseudocode** | **Type** | **Comment** |
|---|---|---|
| SI_search_substrings(si, text) { | | si is a String Index block as specified in Table 4 and text is of type st (v) |
| positions[] = | u(64) [] | String indexing algorithm lookup operation. |
| FM_Index_lookup(si.compressed_index) | | |
| | | This operation returns an array of positions. The returned array may be empty. |
| | | The positions in the returned array are byte-positions within the uncompressed index. |
| return positions[] | | |
| } | | |

### Decoding a sub-set of the String Index

The String Index is decoded between a given start and end positions, inclusive, as specified in the following pseudocode:

**Table 7 - Decoding a substring at a given position with the String Index.**

| **Pseudocode** | **Type** | **Comment** |
|---|---|---|
| SI_decode(si, start, end) { | | si is a String Index block and start and end are of type uint. |
| | | start shall never be greater than end. |
| decoded_payload = | st(v) | String indexing algorithm extract operation which returns the strings comprised between the positions start and end in the original uncompressed concatenation of strings. |
| FM_Index_extract (start, end) | | |
| return decoded_payload | | |
| } | | |

### Searching for whole strings with the String Index

Given a position within the uncompressed index, e.g. one position from the list of positions returned by SI_search_substrings ( ) as specified in this disclosure, the corresponding whole string and its start position within the uncompressed index are decoded with the String Index as specified in the following pseudocode:

**Table 8 - Searching whole strings with the String Index.**

| **Pseudocode** | **Type** | **Comment** |
|---|---|---|
| SI_decode_string(si, pos) { | | si is a String Index block and pos is of type u (64) |
| string = | st (v) | SI_decode() as specified in this disclosure |
| SI_decode(si, pos, pos) | | |
| searching = 1 | uint | |
| for(i = pos - 1; | | |
| i >= 0 && searching; | | |
| i--) { | | |
| ch = SI_decode (si, i, i) | c (1) | SI_decode()as specified in this disclosure |
| chVal = Ord(ch) | uint | Where Ord () returns the numerical ASCII value of ch |
| if(chVal >= 0x20 && chVal <= 0x7F) { | | |
| string = ch + string | | String concatenation |
| } else { | | |
| searching = 0 | | |
| } | | |
| } | | |
| start = i + 1 | uint | |
| searching = 1 | uint | |
| for(i = pos + 1; searching; i++) { | | |
| ch = SI_decode(si, i, i) | c (1) | SI_decode()as specified in this disclosure |
| chVal = Ord(ch) | uint | Where Ord () returns the numerical ASCII value of ch |
| if(chVal >= 0x20 && chVal <= 0x7F) { | | |
| string = string + ch | | String concatenation |
| } else { | | |
| searching = 0 | | |
| } | | |
| } | | |
| return {string, start} | tuple{ st (v), uint } | |
| } | | |

### Searching for Access Unit IDs and Record indexes with the String Index

Given a position, within the uncompressed index, of a byte that belongs to a string encoded in the compressed index, e.g. one position from the list of positions returned by SI_search_subs_trings ( ) as specified in this disclosure, the Access Unit ID of the Access Unit that contains the said string, the index of the Record that contains the said string, and the index of the said string within the said Record are decoded with the String Index as specified in the following pseudocode:

**Table 9 - Searching Access Unit and Record indexes with the String Index.**

| **Pseudocode** | **Type** | **Description** |
|---|---|---|
| SI_decode_string_indexes(si, pos) { | | si is a String Index block as specified in this disclosure, pos is of type uint. |
| auIndex = 0 | uint | |
| while(auIndex < si.num_AUs - 1 | | |
| && | | |
| pos >= si.au_offset[auIndex + 1]) | | |
| { | | |
| auIndex++ | | |
| } | | |
| auIndexOffset = auIndex == 0 | uint | |
| ? 0 | | |
| : si.au_offset[auIndex] | | |
| auId = si.au_id[auIndex] | uint | |
| searching = 1 | uint | |
| for(i = pos - 1; | | |
| i >= auIndexOffset && searching | | |
| i--) { | | |
| ch = SI_decode(si, i, i) | c (1) | SI_decode() as specified in this disclosure |
| chVal = Ord(ch) | uint | Where Ord () returns the numerical ASCII value of ch |
| if(chVal < 0x20 ∥ chVal > 0x7f) { | | |
| searching = 0 | | |
| } | | |
| } | | |
| start = i + 1 | uint | |
| stringIndex = 0 | uint | |
| recordIndexBitPos = 0 | int | |
| recordIndex = 0 | uint | |
| searching = 1 | | |
| for(i = start - 1; | | |
| i >= auIndexOffset && searching; i--) { | | |
| ch = SI_decode(si, i, i) | c (1) | SI_decode() as specified in this disclosure |
| chVal = Ord(ch) | uint | Where Ord () returns the numerical ASCII value of ch |
| if((chVal & 0x80) != 0) { | | |
| chVal = chVal & 0x7f | | |
| recordIndex = recordIndex \| | | |
| (chVal << recordIndexBitPos) | | |
| recordIndexBitPos = | | |
| recordIndexBitPos + 7 | | |
| } else if(recordIndexBitPos > 0) { | | |
| searching = 0 | | |
| } else if(ch == '\n') { | | |
| stringIndex++ | | |
| } | | |
| } | | |
| recordIndex = recordIndex + | | numStrings as specified in this disclosure |
| stringIndex / numStrings | | |
| stringIndex = stringIndex % numStrings | | |
| return {auId, recordIndex, stringIndex} | tuple{ uint, uint, uint | the elements of the result tuple are: |
| | | • auIndex: index, within the Dataset, of the Access Unit containing string, |
| | } | |
| | | • recordIndex: index, within the Access Unit at previous point, of the Record containing string, and |
| | | • stringIndex: index, within the Record at previous point, of string. |
| } | | |

### Searching for the position of the first string of an Access Unit with the String Index

The position, within the uncompressed index, of the first string of a given Access Unit is retrieved with the String Index as specified in the following pseudocode:

**Table 10 - Searching for the position of the first string of an Access Unit with the String Index.**

| **Pseudocode** | **Type** | **Comment** |
|---|---|---|
| SI_au_first_string_pos(si, auIndex) { | | Where si is a String Index block , and auIndex is of type uint and it identifies the Access Unit with ID si.au_id[auIndex] |
| pos = 0 | uint | |
| if (auIndex > 0) { | | |
| pos = si.au_offset[auIndex] | | |
| } | | |
| searching = 1 | uint | |
| for(; searching; pos++) { | | |
| ch = SI_decode(si, pos, pos) | c (1) | SI_decode() as specified in this disclosure |
| chVal = Ord(ch) | uint | Where Ord () returns the numerical ASCII value of ch |
| if(chVal >= 0x20 && chVal <= 0x7F) { | | |
| searching = 0 | | |
| } | | |
| } | | |
| return pos | | |
| } | | |

### Searching for the position of a string of a Record with the String Index

The position, within the uncompressed index, of a string at a given index within a Record, where the Record is at a given index within a given Access Unit, is retrieved with the String Index as specified in the following pseudocode:

**Table 11 - Searching for the position of the first string of a Record with the String Index.**

| **Pseudocode** | **Type** | **Description** |
|---|---|---|
| SI_rec_first_string_pos (si, | | si is a String Index block as specified in this disclosure, auIndex is of type uint and it identifies the Access Unit with ID si.au_id[auIndex], recordIndex is of type uint, stringIndex is of type uint. |
| auIndex, | | |
| recordIndex, | | |
| stringIndex | | |
| ) { | | |
| auStartPos = 0 | uint | |
| auEndPos = (1 << 64) - 1 | uint | |
| if (auIndex > 0) { | | |
| auStartPos = si.au_offset[auIndex] | | |
| } | | |
| if(auIndex < si.num_AUs - 1) { | | |
| auEndPos = si.au_offset[auIndex + 1] | | |
| } | | |
| searching = 1 | uint | |
| currRecordIndex = recordIndex | uint | |
| while(searching && currRecordIndex > 0) { | | |
| marker = "" | st (v) | Empty string |
| i = currRecordIndex | uint | |
| while (i > 0) { | | |
| ch = Chr(i & 0x7F) | c (1) | Where Chr () returns the ASCII character for a given numerical value |
| i = i >> 7 | | |
| marker = ch + marker | | String concatenation |
| } | | |
| marker = "\n" + marker | | String concatenation |
| positions[] = SI_search_substrings (si, marker) | uint[] | List of positions |
| for(i = 0; | | |
| i < Size(positions) && searching; | | |
| i++) { | | |
| pos = positions[i] | uint | |
| if(pos >= auStartPos && pos < auEndPos) { | | |
| pos = pos + Size(marker) | | |
| searching = 0 | | |
| } | | |
| } | | |
| if (searching) { | | |
| currRecordIndex-- | | |
| } | | |
| } | | |
| if (searching) { | | |
| pos = SI_au_first_string_pos(si, auIndex) | uint | SI_au_first_string pos() as specified in this disclosure |
| } | | |
| while(currRecordIndex < recordIndex) { | | |
| count = 0 | uint | |
| while (count < numStrings) { | | numStrings as specified in this disclosure |
| ch = SI_decode(si, pos, pos) | | SI_decode() as specified in this disclosure |
| if(ch == "\n") { | | |
| count++ | | |
| } | | |
| pos++ | | |
| } | | |
| currRecordIndex++ | | |
| } | | |
| count = 0 | | |
| while(count < stringIndex) { | | |
| ch = SI_decode (si, pos, pos) | | SI_decode() as specified in this disclosure |
| if(ch == "\n") { | | |
| count++ | | |
| } | | |
| pos++ | | |
| } | | |
| return pos | | |
| } | | |

### String Index construction

According to the principle of this invention a string index is constructed from textual descriptors using a string transformation method as follows:
- For each annotation separate non-indexed descriptors from indexed textual descriptors
- Concatenate the indexed textual descriptors separated by terminators and interleaved with information on genomic annotation records position within the Access Unit

Numeric descriptors are represented as numerical values and textual descriptors are represented as strings of characters.

In order to compress the resulting string index, the result of the transformation is then further transformed using a compressed full-text string indexing algorithm such as compressed suffix arrays, FM-indexes, and some categories of hash tables.

Interleaving information related to genomic annotation with genomic annotations record positions enables to browse the compressed genomic annotation data according to criteria such as the presence of a string in a record or the genomic interval a genomic record is associated to. Said browsing is performed by specifying textual strings or substrings and retrieving all genomic annotations records containing said text as part of the coded annotation.

An example of an implementation of this construction method is provided in figure 5 where each record contains 3 textual descriptors.

The textual descriptors associated with each genomic annotation type described in this disclosure to build the string index as described above and in figure 5 are selected according to an input configuration encoding parameter provided by the user according to her requirements/needs. This configuration parameter is coded in the bitstream and/or transmitted from the encoder to the decoder.

### Efficient decoding of genomic annotations

By building the compressed string index as described above, it is possible to reconstruct the genomic annotation related to one string descriptor by following the process below.

The goal of this process is to decode all Access Units containing annotation data related to a string identifier specified by a user who is searching for example a variant name or description thereof, genomic feature name or description thereof or any other textual descriptor associated with a coded genomic annotation.

Search the desired name or description by calling the function SI_search_substrings() specified above. If the specified string "str" is present in the compressed index, this call returns one or more positions (named "pos" in this example) as specified in section "Search for substring positions with the String Index". The Access Unit ID of the Access Unit that contains said string "str", the index of the Record that contains said string "str", and the index of said string within the said Record are decoded with the String Index described above in this disclosure as described in the following points:
1. The input byte position "pos" identifies the string str that contains the byte at position pos within the uncompressed index.
2. The ID of the Access Unit that contains str is determined by comparing pos against the values of au_offset[] as specified in Table 4, and retrieving the corresponding value of au_id[] as specified in Table 4,:
   ∘ if pos < au_offset[1], then the resulting Access Unit ID is au_id[0].
   ∘ if pos >= au_offset[num_AUs-1], then the resulting Access Unit ID is au_id[num_AUs-1], with num_AUs as specified in Table 4
   ∘ otherwise the resulting Access Unit ID is au_id[i], for the value of i such that au_offset[i] <= pos < au_offset[i+1].
3. By repeatedly calling function SI_decode() described in this disclosure, decode the compressed index backward from position pos - 1 either until decoding a whole Record Index recordIndex (with Record Index as specified in Table 5) or until reaching the beginning of the compressed index. If the beginning of the compressed index is reached, then the recordIndex is set to 0. While decoding backward, count the number of string terminators recordIndex (with string terminators as specified in Table 5). However any non-printable character can be used as string terminator.
4. Given the number of indexed strings per record numStrings as specified in this disclosure and the Access Unit determined at point 2, the index within the said Access Unit of the Record that contains str is equal to recordIndex + stringIndex / numStrings.
5. Given the number of indexed strings per record numStrings as specified in this disclosure and the Record determined at point 4, the index within the said Record of the string str is equal to stringIndex % numStrings.

### Access Unit

This clause extends the Access Unit syntax specified in ISO/IEC 23092-1 with support of genomic annotations data type encoding.

| **Syntax** | **Key** | **Type** | **Remarks** |
|---|---|---|---|
| *access_unit {* | *aucn* | | |
| access_unit_header | auhd | gen_info | |
| AU_information | auin | gen_info | |
| AU_protection | aupr | gen_info | |
| if (block_header_flag) { | | | |
| for (i=0;i<num_blocks;i++) { | | | |
| block[i] | | | |
| } | | | |
| } | | | |
| } | | | |

### AU Header

| **Syntax** | **Key** | **Type** | **Remarks** |
|---|---|---|---|
| *access_unit_header {* | *auhd* | | |
| access_unit_ID | | uint | |
| num_blocks | | uint | |
| parameter_set_ID | | uint | |
| AU_type | | uint | |
| records_count | | uint | Replaces the reads_count in part 1 |
| if (dataset_type == DS_INTERVAL && AU_TYPE == AU_TRACKS) { | | | |
| track_id | | uint | |
| } | | | |
| if (dataset_type == DS_INTERVAL ∥ dataset_type == DS_CONTACTS) { | | | |
| sequence_ID | | uint | |
| AU_start_position | | uint | |
| AU_end_position | | uint | |
| } | | | |
| if (dataset_type == DS_CONTACTS) { | | | |
| sequence_ID 2 | | uint | |
| AU_start_position_2 | | uint | |
| AU_end_position_2 | | uint | |
| } | | | |
| if (dataset_type == DS_EXPRESSION) { | | | |
| AU_start_feature | | uint | position in the parameter set list of the first feature for which values are stored in the AU |
| AU_feature_count | | uint | Number of consecutive features listed in the parameter set **AU_ features _count <= n_features** |
| } | | | |
| if (dataset_type == DS_EXPRESSION ∥ dataset_type == DS_GENOTYPE) { | | | |
| AU_start_sample | | uint | Index of the first sample coded in the AU in the parameter set list |
| AU_sample_count | | uint | Number of consecutive samples listed in the parameter set **AU_sample_count < = n_samples** |
| } | | | |
| while( !byte_aligned() ) | | | |
| nesting_zero_bit | f(1) | | |
| } | | | |

### Dynamic attributes

1. Most of the genomic annotation formats contain poorly specified fields complementing the minimal set of information defined as compulsory. In some cases, such as VCF, GFF, GTF file formats those fields represent valuable information, since they contain information such as the pathogenicity of a given variant or essential classification clues about elements of functional annotation. Thus, they cannot be simply discarded or treated as secondary information. In fact, some of those field may represent the most valuable filter criteria for clinical purposes.
2. For this reason, all those field, across the several access unit and dataset types described later, are grouped in a set of dynamic attributes. The presence of a given attribute is signaled in the specific section of the parameter set, in object of typ*e "attribute"* specified in this disclosure.
3. Each attribute corresponds to a new descriptor.
4. The presence of a value for a given record is signaled though a record level bitmask, using the position of a given attribute in the parameter set.
5. The attributes are specified in terms of:
   - value type
   - array type, e.g. 1 for if there is a single scalar value, an array of fixed size, an array depending to the number of alleles, poidy or a combination of them, e.g. GL field in genotype columns of a VCF file
   - array size, needed for fixed size arrays

This method provides a unified approach over all the different annotation data types, regardless of their nature, and gives room for future indexing/filtering tools based on the presence of a specific attribute.

| **Syntax** | **Type** | **Description** |
|---|---|---|
| **attributes_parameters(){** | | |
| **n_attributes** | u(8) | |
| **for(a = 0; a < n_attributes; a++){** | | |
| **attributes[a]** | attribute | attributes are data structure specified in this disclosure |
| **}** | | |
| **}** | | |

### Variants

The information on variants is coded in the data structures described in this section, while the information on samples (e.g. genotyping) are coded in a separate dataset.

### Parameters for variants

This structure in the parameters set contains Master parameters related to variant coding.

| **Syntax** | **Type** | **Comment** |
|---|---|---|
| variants_parameters(){ | | |
| n_info | uint | |
| for (i=0 to n_info - 1){ | | |
| info_ID | c(2) | |
| number | uint | |
| type | uint | |
| desc_len | uint | |
| description | c(desc_len) | |
| } | | |
| n_filter | uint | |
| for (i=0 to n_filter){ | | 0 = PASS |
| filter_ID | c(2) | |
| desc_len | uint | |
| description | uint | |
| } | | |
| n_alt | uint | |
| for (i=0 to n_alt){ | | |
| alt_ID | uint | DEL Deletion relative to the reference INS Insertion of novel sequence relative to the reference |
| | | DUP Region of elevated copy number relative to the reference |
| | | INV Inversion of reference sequence |
| | | CNV Copy number variable region (may be both deletion and duplication) |
| | | The CNV category should not be used when a more specific category can be applied. Reserved subtypes include: |
| | | DUP:TANDEM Tandem duplication |
| | | DEL:ME Deletion of mobile element relative to the reference |
| | | INS:ME Insertion of a mobile element relative to the reference |
| desc_len | uint | |
| description | u(desc_len) | |
| } | | |
| n_pedigree | uint | |
| for (i=0 to n_pedigree - 1){ | | |
| pedigree_ID | st(v) | |
| number | uint | |
| keys[number] | st(v) | |
| values[number] | st(v) | |
| } | | |
| pedigreeDB | st(v) | URL to DB |
| vcf_header_flag | uint | |
| if(vcf_header_flag){ | | |
| vcf header | | compressed text of the original VCF header |
| } | | |
| attributes_parameters() | | |
| n_descriptors | uint | number of descriptors used to represent the information of this data type |
| for(n_descriptors) | | |
| descriptor_configuration(i) | | Specific compressor configuration for each descriptor |
| } | | |

### Genomic annotation record for variants

Records shall be sorted per ascending value of pos. Positions are then coded differentially NB: ref_len, ref, alt_len, alt, q_int can be coded as "payload" in the unified record structure; info as attributes.

Genomic annotation records for variants are coded using the common genomic annotation descriptors and the genomic annotation descriptors specific to variants as described in this disclosure.

### Compression of descriptors for variants

Info values are compressed as attributes as described in this disclosure

### ref and alt information

| **SubsequenceID** | **Name** | **Semantics** | **Description** |
|---|---|---|---|
| 0 | parent_ID | | |
| 1 | pos | | Differential encoding with respect to previous record value in the access unit; access unit start position for the first record. The first bit is used to encode the sign, the actual value is then shifted by one bit to the left. |
| 2 | length | | |
| 3 | strand | | |
| 4 | altID | Type of variant | 0 = substitution |
| | | | 1 = DEL |
| | | | 2 = INS |
| | | | 3 = breakends |
| | | | 4 = combination of the above. It must be followed by at least two different values among {0,1,2,3}; the number of the following connected alt is specified in alt_num |
| | | | 5 = element of the alt_list in the parameter set |
| | | | 6 = empty (skip) |
| 5 | alt_num | number of available alternates (ALT in vcf) for REF | It refers to the number of possible alt values e.g.: - if ALT is A,G,T, alt_num =3 |
| | | | always == 1 if altID == 5 |
| 6 | alt | List of alt_num strings | list of ALT values |
| 7 | seqID | identifier of the sequence for breakends. From the contig list in the parameter set | |
| 8 | pos | position value for breakends | |
| 9 | rcomp | reverse complement flag | 0 = sequence is after pos and on the forward strand |
| | | | 1 = sequence is before pos and on the forward strand |
| | | | 2 = sequence is after pos and on the reverse strand |
| | | | 3 = sequence is before pos and on the reverse strand |
| 10 | alt_symbol | List of other polymorphisms | if altID == 5 this contains the position of the symbol in the parameter set list of alt |
| 11 | offset | Offset of the indel when present | When altID == 4, the alt values can be slightly displaced and a subset of them may need a no offset with respect to the declared variant position. |
| | | | e.g. in the case when ref=GCA and alt=GCACA,G, the first alt is an insertion with offset=3, the second alt is a deletion with offset=0 |

### Functional annotations (GTF, GFF)

### Parameters for functional annotations

This structure in the parameters set contains global configuration parameters related to the coding of functional annotations data types.

| **Syntax** | **Type** | **Description** |
|---|---|---|
| **annotation_parameters(){** | | |
| **ontology_name_len** | uint | |
| **ontology_name** | c(ontology_name_len) | Sequence Ontology identifier |
| **ontology_version** | c(16) | version of the ontology used in this coded bitstream |
| **reserved** | u(6) | |
| **n_terms** | u(10) | number of feature types |
| **for(t = 1; t <= n_terms; t++ )** | | 0 reserved for missing feature type name |
| **term_name[t-1]** | st(v) | feature type name max length shall be 33 characters including the terminator |
| **gff_header_flag** | uint | |
| **if(gff_header_flag){** | | |
| **gff_header** | | compressed text of the original GFF header |
| **}** | | |
| **attribute_parameters()** | | |
| **n_descriptors** | uint | number of descriptors used to represent the information of this data type |
| **for(n_descriptors)** | | |
| **descriptor_configuration(i)** | | Specific compressor configuration for each descriptor |
| **}** | | |

### Genomic annotation record for functional annotations

Genomic annotation records for functional annotations are coded using the common genomic annotation descriptors and the genomic annotation descriptors specific to functional annotations as described in this disclosure.

### Compression of descriptors for annotations

| **SubsequenceID** | **Name** | **Description** |
|---|---|---|
| 0 | n_parents | |
| 1 | parent_ID | n_parents elements |
| 2 | pos | Differential encoding with respect to previous record value in the access unit; access unit start position for the first record. The first bit is used to encode the sign, the actual value is then shifted bv one bit to the left. |
| 3 | len | |
| 4 | strand | |
| 5 | type1 | position in parameter set list (0 = missing) |
| 6 | type2 | position in parameter set list (0 = missing) |
| 7 | phase | |
| 8 | score | |

### Tracks

### Parameters for tracks

This structure in the parameters set contains global parameters related to browser tracks coding.

| Syntax | Type | Description |
|---|---|---|
| tracks_parameters(){ | | |
| n_tracks | uint | |
| for (i = 0; i < n_tracks; i++){ | | |
| name_len | uint | |
| name | st(name_len) | |
| desc_len | uint | |
| description | st(desc_len) | |
| tracks_value_type | Type | Type ID can only be 1 or 4 |
| } | | |
| track_header_flag | uint | |
| if(track_header_flag){ | | |
| track_header | | compressed text of the original track header |
| } | | |
| attribute_parameters() | | |
| n_descriptors | uint | number of descriptors used to represent the information of this data type |
| for(n_descriptors) | | |
| descriptor_configuration(i) | | Specific compressor configuration for each descriptor |
| } | | |

### Genomic annotation record for tracks

Genomic annotation records for functional annotations are coded using the common genomic annotation descriptors and the genomic annotation descriptors specific to functional annotations as described in this disclosure.

### Compression of descriptors of tracks

| **SubsequenceID** | **Name** | **Description** | **Example** |
|---|---|---|---|
| 0 | start_pos | bitmask signaling the presence of each attribute | Differential encoding with respect to previous record value in the access unit; access unit start position for the first record. The first bit |
| | | | is used to encode the sign, the actual value is then shifted bv one bit to the left. |
| 1 | len | first attribute values | |
| 2 | strand | second attribute values | |
| 3 | values | as many values as calculated using zoom_level, zoom_span and len | |

### Genotype information

A dataset of type genotype contains coded information related to genotyping information of individuals or populations.

### Parameters for genotype information

This structure in the parameters set contains global configuration parameters related to genotype information coding.

| | | |
|---|---|---|
| genotyping_parameters(){ | | |
| n_format | uint | |
| for (i=0 to n_format - 1){ | | |
| format_ID | c(2) | possible values and semantics are specified in Table 12 Table 12 |
| number | uint | how many values are present |
| type | uint | type of data per value as specified in |
| desc_len | uint | |
| description | uint | |
| } | | |
| } | | |
| genotype_present | b(1) | |
| if(genotype_present){ | | |
| default_ploidy | uint | |
| default_n_alt | | |
| default_phasing | | |
| max_ploidy | uint | |
| default_alt[default_ploidy] | | 0 <= default_alt <= default_n_alt + 1 |
| default_phasing[default_ploidy-1] | b(1) | |
| } | | |
| attribute_parameters() | | |
| n_descriptors | | |
| for(n_descriptors) | | |
| descriptor_configuration(i) | | |
| sample_parameters() | | |
| } | | |

**format_ID** identifies a format field present in the coded records. The semantics of each identifier is provided in Table 12. If the value 0x00 (GT) is present, it shall always be the first in the list.

### Genotype format fields

**Table 12 - format_ID values used in genotype_parameters()**

| format_ID | Field | Number | Type | Description |
|---|---|---|---|---|
| 0 | GT | 1 | String | Genotype |
| 1 | AD | R | Unsigned Integer | Read depth for each allele |
| 2 | ADF | R | Unsigned Integer | Read depth for each allele on the forward strand |
| 3 | ADR | R | Unsigned Integer | Read depth for each allele on the reverse strand |
| 4 | DP | 1 | Unsigned Integer | Read depth |
| 5 | EC | A | Unsigned Integer | Expected alternate allele counts |
| 6 | FT | 1 | String | Filter indicating if this genotype was "called" |
| 7 | GL | G | Float | Genotype likelihoods |
| 8 | GP | G | Float | Genotype posterior probability |
| 9 | GQ | 1 | Unsigned Integer | Conditional genotype quality |
| 10 | HQ | 2 | Unsigned Integer | Haplotype quality |
| 11 | MQ | 1 | Unsigned Integer | RMS mapping quality |
| 12 | PL | G | Signed Integer | Phred-scaled genotype likelihoods rounded to the closest integer |
| 13 | PQ | 1 | Unsigned Integer | Phasing quality |
| 14 | PS | 1 | Unsigned Integer | Phasing set |
| 15..255 | | | | reserved |

| | | | | |
|---|---|---|---|---|
| A = one value per alternate allele R = one value for each possible allele including the reference G = one value per genotype | | | | |

### Genomic annotation record for genotype information

Genomic annotation records for genotype information are coded using the common genomic annotation descriptors and the genomic annotation descriptors specific to genotype information as described in this disclosure.

### Compression of genotype information

All the information is compressed as attributes, as described in this disclosure. Special cases, such as GT and LD fields, are first split in subsequences identified by subsequenceID as described below.

| **Format value** | **SubsequenceID** | **Name** | **Semantics** | **Type** | **Description** |
|---|---|---|---|---|---|
| | 0 | bitmask[n_format] | n_format bits signalling the presence of each format field | **bit** | |
| 0 | 1 | default_gt | flag signaling if the genotyping information is equal to the default one in the parameter set (0) or it is coded here (1) | | |
| | 2 | default_ploidy | 0 = is default value | **bit** | |
| | | | 1 = value is in subseq 2 | | |
| | 3 | ploidy | ploidy when subseq 1 == 1 | **uint** | |
| | 4 | phasing[size] | size is default ploidy -1 if default_ploidy == 0 else size is ploidy - 1 | **bit** | Consider a default for the AU |
| | | | 0 == phased | | |
| | | | 1 == unphased | | |
| | 5 | alt_gt[ploidy] | A tuple of length ploidy of alt, each of size u(ceil(log2(alt_len+1))) | | |
| 1 | 6 | value[n_alt+1] | | uint | AD |
| | | | | | Read depth for each allele |
| 2 | 7 | value[n_alt+1] | | uint | ADF |
| | | | | | Read depth for each allele on the forward strand |
| 3 | 8 | value[n_alt+1] | | uint | ADR |
| | | | | | Read depth for each allele on the reverse strand |
| 4 | 9 | value | | uint | DP |
| | | | | | Read depth |
| 5 | 10 | value[n_alt] | | uint | EC |
| | | | | | Expected alternate allele counts |
| 6 | 11 | filter | | string | FT |
| | | | | | Filter indicating if this genotype was "called" |
| 7 | 12 | is_default_comma | 0 = the position of the comma is the default one | | Genotype likelihoods |
| | | | 1 = the position of the comma is in subseq 3 | | |
| | 13 | value | the value as an integer | | |
| | 14 | comma_pos | comma position if not default | | |
| 8 | | | | | Genotype posterior probability |

### Sample information

### Parameters for sample information

This structure in the parameters set contains global configuration parameters related to the coding of information about samples.

| | | |
|---|---|---|
| **sample_parameters(){** | | |
| **n_meta** | uint | TBD, table with correspondence code->tag |
| **for (i=0 to n_meta - 1){** | | |
| **meta_ID** | uint | |
| **number** | uint | |
| **type** | uint | |
| **values[number]** | **type** | list of allowed values |
| **}** | | |
| **}** | | |
| **attribute_parameters()** | | |
| **n_descriptors** | | |
| **for(n_descriptors)** | | |
| **descriptor_configuration(i)** | | |
| **sample_parameters()** | | |
| **}** | | |

### Genomic annotation record for samples information

Genomic annotation records for samples information are coded using the genomic annotation descriptors specific to samples information as described in this disclosure.

### Expression information

This dataset codes only the actual expression matrix. The features are stored in access unit of type AU_ANNOTATION and the samples in access units of type AU_SAMPLE.

### Expression parameters

This structure in the parameters set contains global configuration parameters related to the coding of expression information.

| | | |
|---|---|---|
| **expression_parameters(){** | | |
| **sample_parameters()** | | |
| **n_format** | uint | |
| **for (i=0 to n_format - 1){** | | |
| **format_ID** | c(2) | As used in matrix headers |
| **number** | uint | how many values are present |
| **type** | value_type | type of data per value as specified in |
| **desc_len** | uint | |
| **description** | u(desc_len) | |
| **}** | | |
| **n_features** | uint | |
| **for(n_features){** | | Ordered list of features. For indexing, each feature shall be identified by its position in this list. |
| **feature_name** | st(v) | **Compressed using a string indexing algorithm** |
| **}** | | |
| **attribute_parameters()** | | |
| **n_descriptors** | uint | number of descriptors used to represent the information of this data type |
| **for(n_descriptors)** | | |
| **descriptor_configuration(i)** | | Specific compressor configuration for each descriptor |

**format_ID** identifies a format field present in the coded records. The semantics of each identifier is provided in Table 12. (table 12)

### Genomic annotation record for expression information

Genomic annotation records for expression information are coded using the genomic annotation descriptors specific to expression information as described in this disclosure.

### Compression

The compression strategy is the same as for the Genotype datasets: all the information are mapped into attributes and compressed, as described in the section titled "Compression of Attributes". This allows to have more than one value for each element of the matrix, thus combining in a single record information such as counts, tpm, probabilities etc., with different types and semantics.

A special approach is used for sparse matrices, where, for each record, only the non-zero values are recorded, together with an array of the corresponding positions and the total number of entries.

### Contact matrices information

Contact matrices (a.k.a. contact maps) are generated by Hi-C experiments and represent the spatial organization of a DNA molecule in the cell nucleus. The two dimensions are genomic positions. The contact matrix value at each coordinate represent a counter of how many times the two positions in the nucleotide sequences have been measured to have an interaction.

### Contacts parameters

This structure in the parameters set contains global configuration parameters related to the coding of information on contact matrices.

| **syntax** | **data type** | **description** |
|---|---|---|
| **contacts_parameters(){** | | |
| **n_format** | uint | |
| **for (i=0 to n_format - 1){** | | |
| **number** | uint | how many values are present |
| **type** | value_type | type of data per value as specified in this disclosure. |
| **desc_len** | uint | |
| **description** | u(desc_len) | |
| **}** | | |
| **n_descriptors** | uint | number of descriptors used to represent the information of this data type |
| **for(n_descriptors)** | | |
| **descriptor_configuration(i)** | | Specific compressor configuration for each descriptor |
| **}** | | |

**format_ID** identifies a format field present in the coded records. The semantics of each identifier is provided in Table 12 (Table 12)

### Genomic annotation record for contact matrices information

Genomic annotation records for samples information are coded using the genomic annotation descriptors specific to sample information as described in this disclosure.

### Compression

The compression strategy is the same as for the Expression information datasets.

### Attributes

| **Syntax** | **Type** | **Description** |
|---|---|---|
| **attribute{** | | |
| **attribute_name** | st(v) | Attribute identifier |
| **attribute_array_type** | array_type | Scalar or array and corresponding size policies, see description of array types below. |
| **attribute_size** | uint | How many values per attribute; needed for fixed size arrais, zero for all the other values of array_type |
| **attribute_type** | value_type | Type of data as defined in this disclosure |
| **}** | | |

### Compression of Attributes

Attributes are compressed using as many subsequences as n_attributes in the parameter set + 1

| SubsequenceID | Name | Description | Example |
|---|---|---|---|
| 0 | attr_mask | bitmask signaling the presence of each attribute | |
| 1 | attr1 | first attribute values | |
| 2 | attr2 | second attribute values | |
| ... | | | |
| n | attrn | n^{th} attribute values | |

### Data types

This sections describes how structured values are represented in this disclosure.

### Value type

This is a structure used to represent numerical values with their sizes in bits.

| | | |
|---|---|---|
| **value_type{** | | |
| **type_ID** | uint | as per Table 13 (Table 13) |
| **if(type_ID == 1)** | | |
| **type_size** | uint | |
| **else if(type_ID == 2)** | | |
| **type_size** | uint | |
| **else if(type_ID == 3)** | | |
| **n_characters** | uint | |
| **else if(type_ID == 4){** | | |
| **integer_size** | uint | number of digits in the integer part |
| **decimal_size** | uint | number of digits in the fractional part |
| **}** | | |
| } | | |

### Type identifiers

**Table 13 - Data types with their identifiers and parameters**

| **type_ID** | **type** | **parameters** |
|---|---|---|
| 0 | bool | |
| 1 | uint | size in bits u(6) |
| 2 | int | size in bits u(7) |
| 3 | string | size in characters u(10) |
| 4 | decimal/numeric | u(4), u(5) |
| | | /* e.g. database like with number of total digits and number of decimal digits */ |
| 5 | float32 (IEEE 754) | |

### Array identifiers

**Table 14: Array types with their identifiers**

| **array_type_ID** | **Corresponding array size** |
|---|---|
| 0 | Scalar, e.g. only one value |
| 1 | Fixed array size |
| 2 | Array of length equal to the number of alternate alleles |
| 3 | Array of length equal to the total number of alleles plus reference |
| 4 | genotype-likelihood field: its size depends on the combination of the total number of alleles and the ploidy |

### Data Block

Data blocks are structures containing the compressed descriptors and encapsulated in Access Units. Each block contains descriptors of a single type which is identified by an identifier contained in the block header

### Block Syntax

| **Syntax** | **Type** |
|---|---|
| block() { | |
| block_header() | block header |
| block_payload() | block payload |
| } | |

### Block Header

| **Syntax** | **Type** |
|---|---|
| block_header() { | |
| **reserved** | uint |
| **descriptor_ID** | uint |
| **reserved** | uint |
| **block_payload_size** | uint |
| } | |

### Block Payload

| **Syntax** | **Type** |
|---|---|
| block_payload(descriptor_ID) { | |
| if(descriptor_ID == 11 ∥ descriptor_ID == 15){ | |
| encoded_tokentype() | |
| } | |
| else { | |
| encoded_descriptor_sequences(descriptor_ID) | compressed descriptor sequences |
| } | |
| while( !byte_aligned( ) ) | |
| **nesting_zero_bit** | f(1) |
| } | |

### Examples of supported queries

| ID | Input parameters | Output | |
|---|---|---|---|
| 1 | Genomic interval (or position) and (optionally) feature type | • Functional annotation for that interval. That is usually expressed as a list of genes; to each gene a list of transcript is associated; depending on its nature, each transcript is made of a set of one or more exons/introns, 5'/3' untranslated regions (UTRs), start/stop codons, etc. Functional annotation can include the sequence of the transcripts and/or proteins produced by the different spliceforms | |
| | | • Expression of genes being contained in the specified interval, for all the samples being associated with the specified gene | |
| | | • Variants, and related information, included in the interval. **If** there is sample information associated with the variant, the list of samples in which the variant is present | |
| | | • Genotyping information for genomic positions contained in the interval | |
| | | • **If** one or more signal tracks (associations between genomic positions and a value, such as coverage at that position for some experiment, for instance DNA-, RNA-, or ChIP-sequencing), the values of each track at | |
| | | | all the positions of the specified interval. Different track resolutions can be made available for each track (use case of a genome browser at different zooming levels) |
| | | **•** | If sequencing reads are present in the specified interval, a list ("pileup") containing for each read sequence, qualities, and any other information possibly associated with each read. |
| | | In case a feature type is specified, the output just described is filtered in order to only retrieve the desired type of feature. Thanks to the different features being compressed separately, that can be achieved by performing a selective decompression of only part of the data | |
| 2 | Textual string and (optionally) feature type | All the features mentioned above (functional annotations, expression, variants) for which either the unique name of the feature or some associated textual description field which has been indexed in the MSI contains the string specified in the query. If a specific feature type is queried, only information pertaining to that type of feature is retrieved. Thanks to the different features being compressed separately, that can be achieved by performing a selective decompression of only part of the data | |
| 3 | Variant name | In addition to the outputs mentioned in (2) when the feature type is "variant": | |
| | | • List of all the samples containing the variant | |
| | | • Associated metadata for each sample | |
| 4 | Sample name | • List of all the (gene) expression values associated with that sample | |
| 5 | Gene name | • List of all the expression values, and the corresponding sample names, associated with that gene | |
| 6 | Genomic intervals A and B | • If links between any positions in A and any positions in B have been found via, for instance, Hi-C experiment, the list of such connections. Different binning levels can be made available for each contact matrix. | |

### Evidence of technical advantage of present invention

The present invention removes a number of problems present when using state of the art technologies. In particular:
1. At the moment, no unified representation of genomic annotations exists. Instead, a number of heterogeneous formats are used. Usually it is implicitly assumed that features are connected according to their physical proximity on the genome, i.e., for instance, a variant or an isoform are related to their containing gene. The unified representation of data described in this invention make possible to express complex relations between different concepts even beyond simple physical containment, such as "The promoter located at this interval, and its methylation state (which are usually external to genes) are related with gene A, gene B and gene C, which form an operon (i.e. a collection of genes each one having a different position in the genome)"
2. The present invention make possible to explicitly connect with the existing parts 1-5 of the MPEG-G standard, where sequencing reads aligned to the genome are represented. Many of the annotated features (such as functional gene models, variants or tracks expressing, for instance, methylation states or binding to proteins) are supported by, and sometimes derived from, the presence of sequencing reads at the relevant locations. Currently it is not possible to express concepts such as "This new transcript, which is made of this list of exons, is supported by this set of RNA-sequencing reads" or "This new variant, located at this position, is supported by this set of DNA-sequencing reads". The present invention make possible to express these concepts (the latter being very important in clinical practice) effortlessly
3. At the moment there is no single format able to represent all the different existing sources of genomic annotations. As a result, pipelines and genome browsers need to use a number of different formats in order to load all the needed information. The present inventions removes the technical need to implement complex parsers for such domain-specific bioinformatics formats, which are often ill-defined and lacking a defined standard
4. Thanks to the separation of information into different types of Access Units, the present invention provides for a mechanism to implement efficient compression - each information stream can be modelled as a homogeneous source having lower entropy, thus making compression more efficient. On the other hand, the proposed method still allows integration of different information into a single hierarchical architecture, and the possibility of expressing relations between different genomic annotation concepts, genomic sequences and sequencing reads. In addition, having different genomic features compressed separately allows selective decompression of the desired feature should the user only be interested in a subset of the data
5. The adoption of a set of compressed string index algorithms, from which one algorithm can be chosen at encode time, to compress textual information, allows the user to select the desired balance between compression of the string index and speed when querying it. Notably, the use of more than one family of compressed string index algorithms is essential to achieve the desired optimizations and an essential feature of the present invention, as the adoption of one single family would not be sufficient for the purpose.

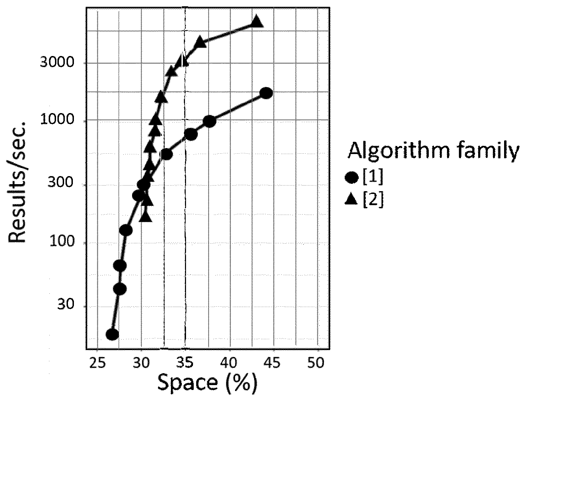

As an example but not as a limitation, we illustrate the concept by combining two different families of compressed suffix arrays. Family [1] uses bitvectors implemented as described in Raman, Rajeev, Venkatesh Raman, and S. Srinivasa Rao. 2002. "Succinct indexable dictionaries with applications to encoding k-ary trees and multisets." In Proceedings of the 13th Annual ACM-SIAM Symposium on Discrete Algorithms (SODA 2002), 233-242. Family [2] uses bitvectors implemented as described in Juha Kärkkäinen, Dominik Kempa, Simon J. Puglisi. Hybrid Compression of Bitvectors for the FM-Index. In Proc. 2014 Data Compression Conference (DCC 2014), IEEE Computer Society, 2014, pp. 302-311. As shown in the figure 6, it is possible to change other parameters of the compressed suffix array families in order to obtain different compressed suffix array implementations that belong to family [1] (pink dots) and family [2] (cyan dots) and show different values for compression rate and querying speed. However, family [1] is inherently better at providing higher compression rates (and slower querying speeds) while family [2] is inherently better at providing faster querying speeds (and lower compression rates). By combining the two families, and selecting as set of possible compressed suffix arrays the ones identified by the black rectangles, we are able to provide choices with better compression rate and choices with better querying speed, which would be impossible by just using one family of compressed suffix arrays.

### Indexing capabilities

| **ID** | **Use case** | **Input parameters** | **Output** | **Test items** |
|---|---|---|---|---|
| 1 | Variant calling on single individual | genomic interval/position | variants information, tracks values, genome features hierarchy (gene, transcripts, exons, introns) | VCF BigWig GFF3 |
| 2 | Large variants database | genomic interval/position | variants information | VCF |
| 3 | RNAseq | genomic interval | all expressions of all genes in that interval in all samples | MatrixMarket + tsv with samples and features |
| 4 | RNAseq | gene name | coverage, expression of that gene in all samples | BigWig MatrixMarket + tsv with samples and features |
| 5 | Population Genetics | variant position and genotype | all datasets with that variant (description, metadata) | VCF with samples |
| 6 | variant by identifier | variant identifier | variants information, tracks values, genome features hierarchy (gene, transcripts, exons, introns) | VCF, BigWig, GFF3 |
| 7 | search for text | comment in annotation | all records containing the comment | GFF3, VCF |
| 8 | search for gene info | gene name | variants information, tracks values, genome features hierarchy (gene, transcripts, exons, introns) | VCF, BigWig, GFF3 |
| 9 | gene expression | gene name | all expressions of all genes with this name in all samples | MatrixMarket + tsv with samples and features |
| 10 | search for annotation type | annotation type | list of all features of that type | GFF3 |
| 11 | extract contact sub-matrix | pair of genomic intervals | sub-matrix with contact values | Hi-C |
| 12 | search for contact regions | genomic interval | list of contact values and the corresponding locations for contact values over a given threshold within the specified genomic interval | Hi-C |

### Genomic annotations encoding apparatus

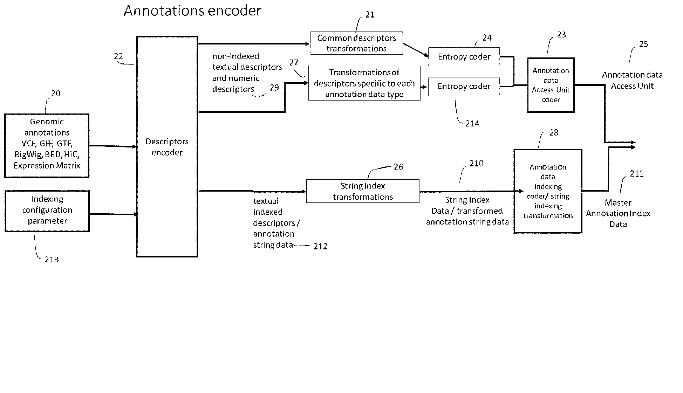

Figure 2 shows an encoding apparatus according to the principles of this invention. The encoding apparatus receives as input genomic annotations such as variants, browser tracks, functional annotations, methylation patterns and levels, sequencing coverage and statistics, feature expression matrices, contact matrices, affinity of a protein for nucleic acids, 20. The annotation data is parsed by a descriptors encoder unit 22 and non-indexed descriptors are separated from textual indexed descriptors 212. Non-indexed descriptors common to all annotations are fed to a transformation unit 21. Non-indexed descriptors specific to each annotation type are fed to a transformation unit 27. Textual indexed descriptors are fed to a descriptors string transformation unit 26. The outputs of transformation units 21 and 27 are fed to different entropy coders 24 according to the specific statistical properties of each transformed descriptors. At least one first entropy encoder (24) is employed for the numeric descriptors and at least one second entropy encoder (214) is employed for the textual descriptors not included in said subset of textual descriptors (29).

The output of each entropy coder is fed to an Annotation Data Access Unit coder 23 to produce Annotation data Access Units 25. The Uncompressed Master Annotation Index 210, output of the descriptor string index transformation unit 26 is fed to an Annotation data indexing coder 28 to produce Master Annotation Index Data 29. One annotation data index is associated with one or more Annotation data Access Units. Figure 1 shows that annotation data Access Units (122) are jointly coded (118) with the Master Annotation Index Data (123) and the Access Units of the first sort (119) containing compressed genome sequencing data.

The transformations applied by the descriptors transformation units 21 and 27 used in the encoding apparatus include:
∘ run-length coding: sequences of numbers are represented by a counter of consecutive occurrences and the values of the occurrences
∘ differential coding: each number is represented as difference with respect to the previously coded value
∘ bytes separation: for numbers represented by a multiplicity of bytes, each byte is processed separately and compressed with other bytes having similar properties in terms of bits configuration

The transformations applied by the annotation data indexing coder 28 include:
∘ Burrows Wheeler Transform
∘ compressed string pattern matching
∘ compressed suffix arrays,
∘ FM-indexes
∘ hashing algorithms

The advantages of applying said transformation to numerical descriptors is to improve compression efficiency without loss of information as it is known to any person skilled in the art.

Coding of string descriptors is made more efficient by said transformation as the transformed representation is more efficiently browsable and searchable for sub-strings. Once the original text is transformed, the presence of sub-strings can be verified without decompressing the whole text.

### Genomic annotations decoding apparatus

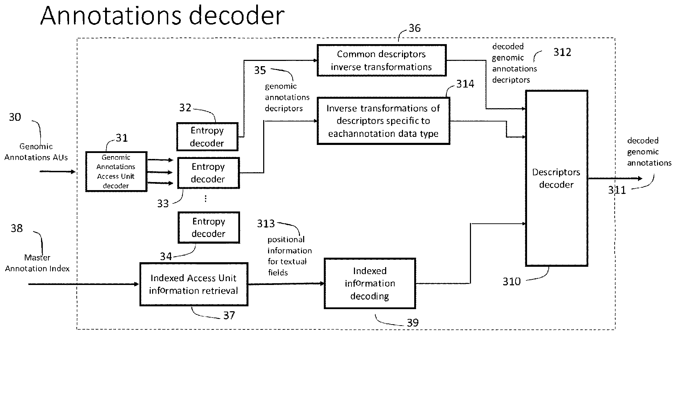

A decoding apparatus implemented according to the principles of this disclosure extends the functionality of a decoding apparatus compliant with ISO/IEC 23092 as depicted in figure 3.

Figure 3 shows a decoding apparatus according to the principles of this disclosure. A genomic annotations Access Units decoder 31 receives Access Units 30 from a stream demultiplexer 70 and extracts the entropy coded payload of the Access Units. Entropy decoders 32, 33, 34 receive the payloads extracted which are entropy coded and decode the different types of genomic annotation descriptors into their binary representations 35. Said binary representations of common descriptors to all genomic annotations are then fed to an inverse transformation unit 36. Binary representations of descriptors specific to each annotation data type are fed to an inverse transformation unit 314. A Master Annotation Index 38 is fed to an Indexed Access Unit information retrieval unit 37 which locates in the string index the textual fields belonging to each AUs. Such positional information 313 is then fed to an Indexed information decoding unit 39 which decodes the textual fields from the string index. Said decoded textual fields are then fed to a descriptors decoder unit 310 to reconstruct the decoded genomic annotations 311.

### Genomic annotations textual search apparatus

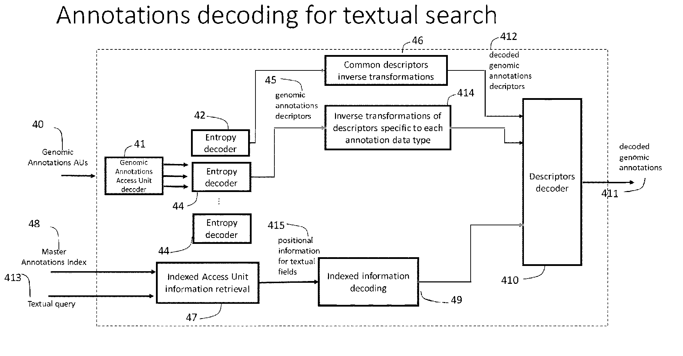

A textual search apparatus implemented according to the principles of this disclosure extends the functionality of a decoding apparatus compliant with ISO/IEC 23092 as depicted in figure 4.

Figure 4 shows a decoding apparatus according to the principles of this disclosure. A genomic annotations Access Units decoder 41 receives Access Units 40 from a stream demultiplexer 70 and extracts the entropy coded payload of the Access Units. Entropy decoders 42, 43, 44 receive the payloads extracted which are entropy coded and decode the different types of genomic annotation descriptors into their binary representations 45. In a configuration of the decoding apparatus, the Access Units of different types or different sorts can be selectively extracted. Said binary representations of common descriptors to all genomic annotations are then fed to an inverse transformation unit 46. Binary representations of descriptors specific to the annotation data type are fed to an inverse transformation unit 414. A Master Annotations Index 48 is fed to an Indexed Access Unit information retrieval unit 47 which locates in the string index the textual fields matching a textual query 413. Such positional information 415 is then fed to an Indexed information decoding unit 49 which decodes the textual fields from the string index. Said decoded textual fields are then fed to a descriptors decoder unit 410 to reconstruct the decoded genomic annotations 411.

The inventive techniques herewith disclosed may be implemented in hardware, software, firmware or any combination thereof. When implemented in software, these may be stored on a computer medium and executed by a hardware processing unit. The hardware processing unit may comprise one or more processors, digital signal processors, general purpose microprocessors, application specific integrated circuits or other discrete logic circuitry.

The techniques of this disclosure may be implemented in a variety of devices or apparatuses, including mobile phones, desktop computers, servers, tablets and similar devices.

## Claims

1. A computer-implemented method for
the storage or transmission of a representation of genome sequencing data in a genomic file format comprising annotation data associated with said genome sequencing data, said genome sequencing data comprising reads of sequences of nucleotides, said method comprising the steps of:
aligning (10) said reads to one or more reference sequences thereby creating aligned reads,
classifying (14) said aligned reads according to classification rules based on mapping of said aligned reads on said one or more reference sequences, thereby creating classes of aligned reads (18),
entropy encoding said classified aligned reads as a multiplicity of blocks of descriptors,
structuring said blocks of descriptors with header information thereby creating Access Units of a first sort (119) containing genome sequencing data,
said method further comprising encoding annotation data (12) into different Access Units of a second sort (122) and indexing data into a master annotation index (MAI, 123, 211), wherein said indexing data represent an encoded form of annotation string data obtained by employing at least one compressed string indexing algorithm (28) on said annotation string data (212), and wherein said MAI associates encoded annotation strings with said access units of a second sort,
said method further comprising jointly coding said access units of first sort, of second sort and said MAI.

2. The method of claim 1, wherein said access units of the second sort containing genomic annotation data further comprise information data identifying a genomic interval (80), wherein said genomic interval identifies a sequence of nucleotides in the one or more reference sequences such that the annotation data contained in the access units of the second sort are associated with the related encoded reads of the genomic sequence contained in access units of the first sort containing genome sequencing data.

3. The method of claim 2, wherein the encoding of said annotation data and indexing data comprises the steps of:
encoding (22) genomic annotation data (20) as genomic annotation descriptors (29, 212), wherein said genomic annotation descriptors comprise numeric descriptors and textual descriptors, said encoding comprising the steps of :
- selecting a subset of textual descriptors (212) from said textual descriptors according to a configuration parameter (213), in particular provided by the user;
- transforming (26) said subset of textual descriptors (212) by employing a first string transformation method to produce a string index (210);
- transforming and encoding (28) said string index (210) by employing a string indexing transformation method thereby producing master annotation index data (211);
- transforming (21, 27) said numeric descriptors and the textual descriptors not included in said subset of textual descriptors (29) by employing at least one second transformation method (21, 27) different from the first transformation method;
- encoding (24, 23) said numeric descriptors and the textual descriptors not included in said subset of textual descriptors (29) into separate access units (25) of the second sort, by employing at least one first entropy encoder (24) for the numeric descriptors and at least one second entropy encoder (214) for the textual descriptors not included in said subset of textual descriptors (29).

4. The method of claim 3, wherein said first string transformation method (26) comprises the steps of:
- inserting a string terminator (55) character for signaling the termination of each textual descriptor (51, 52, 53), after each textual descriptor (51, 52, 53);
- concatenating the textual descriptors (51, 52, 53);
- interleaving genomic annotation record index data (54) for associating said textual descriptors (51, 52, 53) with the position of a genomic annotation record within the Access Unit of the second sort.

5. The method of claim 4, wherein the string indexing transformation method (28) is one of string pattern matching, suffix arrays, FM-indexes, hash tables.

6. The method of claim 3, wherein said at least one second transformation method (21, 27) is one of: differential coding, run-length coding, bytes separation, and entropy coders such as CABAC, Huffman Coding, arithmetic coding, range coding.

7. The method of any of the preceding claims, wherein said master annotation index (MAI) contains in its header the number of AU types and the number of indexes for each AU type.

8. The method of any of the preceding claims, further comprising coding of classified unaligned reads.

9. A method for the decoding and extraction of sequences of nucleotides and genomic annotations data encoded according to the method of claim 1, said method comprising the steps of:
parsing (70) a genomic data multiplex (710) into genomic layers (71) of syntax elements;
parsing compressed annotation data (712);
parsing a master annotation index (MAI) (713);
expanding said genomic layers into classified reads of sequences of nucleotides;
selectively decoding said classified reads of sequences of nucleotides on one or more reference sequences so as to produce uncompressed reads of sequences of nucleotides;
selectively decoding said annotation data associated with said classified reads.

10. The method of claim 9 further comprising decoding information data related to a genomic interval (80), wherein said genomic interval identifies a sequence of nucleotides in the one or more reference sequences such that the annotation data are associated with the related encoded reads of the genomic sequence.

11. The method of claim 10 further comprising decoding the data encoded according to claims 3-8.

12. A genomic encoder (110) for the compression of genome sequence data in a genomic file format comprising annotation data associated with said genome sequencing data, said genome sequence data comprising reads of sequences of nucleotides, said encoder comprising:
- an aligning unit (10) for aligning said reads to one or more reference sequences thereby creating aligned reads;
- a data classification unit for classifying (14) said aligned reads according to classification rules based on mapping of said aligned reads on said one or more reference sequences, thereby creating classes of aligned reads,
- entropy coding units (112) for entropy encoding said classified aligned reads as a multiplicity of blocks of descriptors (115),
- an access unit coding unit (116) for structuring said blocks of descriptors with header information thereby creating Access Units of a first sort (119,219) containing genome sequencing data,
- a genomic annotation encoding unit (117) for encoding annotation data (12) into different Access Units of a second sort (122) and indexing data into a master annotation index (MAI, 123), wherein said indexing data represent an encoded form of annotation string data obtained by employing at least one compressed string indexing algorithm (28) on said annotation string data (210), and wherein said MAI associates encoded annotation strings with said access units of a second sort.
- means for jointly coding said access units of first sort, of second sort and said MAI.

13. The genomic encoder of claim 12 further comprising encoding means for performing the steps of the encoding method of claims 1-8.

14. A genomic decoder apparatus for the decoding of sequences of nucleotides and genomic annotations data encoded by the encoder of claim 12, said decoder comprising:
- means for parsing (70) a genomic data multiplex (710) into genomic layers (71) of syntax elements,;
- means for parsing said compressed annotation data;
- means for parsing a master annotation index;
- means for expanding said genomic layers into classified reads of sequences of nucleotides;
- means for selectively decoding said classified reads of sequences of nucleotides on one or more reference sequences so as to produce uncompressed reads of sequences of nucleotides;
- means for selectively decoding said annotation data associated to said classified reads.

15. The genomic decoder of claim 14 further comprising decoding means for performing the steps of the decoding method of claims 10-11.

16. A computer-readable medium comprising instructions that when executed by at least one processor, cause the at least one processor to perform the method of any one of the claims 1 to 11.
